(19) Europäisches Patentamt
European Patent Office
Office européen des brevets

(11) EP 0 773 949 B1

(12) **FASCICULE DE BREVET EUROPEEN**

(45) Date de publication et mention de la délivrance du brevet:
**10.11.1999 Bulletin 1999/45**

(21) Numéro de dépôt: **95922576.4**

(22) Date de dépôt: **07.06.1995**

(51) Int Cl.[6]: **C07H 13/04**, A61K 31/70, A61K 31/34, A61K 31/215, C07D 317/24, C07C 69/33, C07C 69/612

(86) Numéro de dépôt international:
**PCT/FR95/00743**

(87) Numéro de publication internationale:
**WO 96/03411 (08.02.1996 Gazette 1996/07)**

# (54) ESTERS DU D-MANNOSE OU DU XYLITOL ET LEUR UTILISATION COMME MEDICAMENTS

D-MANNOSE- ODER XYLITO-ESTER UND IHRE VERWENUNG ALS ARZNEIMITTEL

D-MANNOSE OR XYLITOL ESTERS AND THEIR USE AS DRUGS

(84) Etats contractants désignés:
**AT BE CH DE DK ES FR GB GR IE IT LI LU MC NL PT SE**

(30) Priorité: **28.07.1994 FR 9409348**

(43) Date de publication de la demande:
**21.05.1997 Bulletin 1997/21**

(73) Titulaire: **L'ASSOCIAZIONE DI VOLONTARIATO "PRO LA FONDAZIONE FUTURO SENZA THALASSEMIA"**
**50129 Florence (IT)**

(72) Inventeurs:
- **BALDWIN, Pierre-Jean**
  **F-80160 Prouzel (FR)**
- **DOUILLET, Olivier, Claude, Eric**
  **F-80370 Agenville (FR)**
- **POUILLART, Philippe, René, Michel**
  **F-80330 Longueau (FR)**
- **RONCO, Gino, Lino**
  **F-80000 Amiens (FR)**
- **VILLA, Pierre, Joseph**
  **F-80000 Amiens (FR)**

(74) Mandataire: **Bernasconi, Jean et al**
**c/o Cabinet Lavoix,**
**2, Place d'Estienne d'Orves**
**75441 Paris Cédex (FR)**

(56) Documents cités:

| | |
|---|---|
| **WO-A-89/02895** | **FR-A- 2 531 632** |
| **GB-A- 2 128 613** | **US-A- 3 053 677** |

- **CHEMICAL ABSTRACTS, vol. 075, no. 20, 15 Novembre 1971, Columbus, Ohio, US; abstract no. 119600, DANILOV S N ET AL 'Flotation properties of xylitol esters' & ZH. PRIKL. KHIM. (LENINGRAD) (ZPKHAB);71; VOL.44 (6); PP.1342-7, INST. VYSOKOMOL. SOEDIN.;KOL'SK. FIL.; USSR**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 773 949 B1

Printed by Jouve, 75001 PARIS (FR)

## Description

La présente invention concerne des esters associant les acides phénylacétique, 3-phénylpropionique, 4-phénylbutyrique ou n-butyrique au D-mannose, au xylitol et à leurs dérivés, ainsi que leurs applications comme médicaments.

Il est connu que les acides phénylacétique, 4-phénylbutyrique et n-butyrique et leurs sels stimulent la synthèse de l'hémoglobine foetale, notamment celle de la chaine γ (E. FIBACH et coll., Blood (1993), 82 (7), 2203 ; S.P. PERRINE et coll., Biochem. Biophys. Res. Comm. (1987), 148 694). De plus ils induisent le gène γ de l'hémoglobine foetale humaine (J.W. ZHANG et coll., Developmental Genetics (1990), 11 168 ; J.G. GLAUBER et coll., Molec. Cell. Biol. (1991), 11, 4690). Ces propriétés ont été mises à profit pour traiter des patients atteints d'anémie et de syndromes β-thalassémiques (S.P. PERRINE et coll., N. Engl. J. Med. (1993), 328 (2), 81).

Il est connu également que ces acides et leurs sels inhibent la croissance et induisent la différenciation de cellules prémalignes et malignes (D. SAMID et coll., Cancer Res. (1992), 52 1988; E. GINSBURG et coll., Proc. Natl. Acad. Sci. (1973), 70 2457 ; K. YAMADA et coll., J. Cell. Physiol. (1985), 125 235 ; K.N. PRASAD, Life Sciences (1980), 27 1351), notamment de cellules leucémiques (S. FISCKHOFF et coll., Leukemia (1990), 4, 302 ; D. SAMID et coll., Cancer Res. (1992), 52 1988).

Il est connu également que l'acide *n*-butyrique et ses sels ont une durée de vie plasmatique *in vivo* très brève (P. DANIEL et coll., Clin. Chim. Acta (1989), 81 255). Il est connu également que des esters résultant de l'association par liaison covalente de cet acide au D-glucose, au D-galactose, au glycérol et à leurs dérivés sont susceptibles, *in vivo*, sous l'action des systèmes enzymatiques chez l'Homme ou l'Animal, de libérer lentement l'acide n-butyrique avec comme résultat une durée de vie plasmatique beaucoup plus longue, procurant ainsi une meilleure biodisponibilité de la partie biologiquement active (F. PIERI et coll., Brevet FR 871294 (1987) ; Brevet FR 8809092 (1988) ; PCT/FR 88/00470 (1988) ; Brevet U.S. 071501 (1990)).

Il est connu également, par les études biologiques des esters butyriques précédemment cités, que le composé le meilleur comme médicament, est le monoeste 3-O-*n*-butanoyl-1,2-O-isopropylidène-α-D-glucofuranose (P. POUILLARD, Thèse, Amiens, 1990 ; P. PLANCHON, Thèse, Amiens, 1991). Cependant ce composé, du fait de sa structure présentant 2 groupements hydroxylés OH voisins du groupement O-*n*-butanoyle, subit une transestérification interne conduisant, en solution, au mélange des 3 isomères 3-O-butanoyle, 5-O-butanoyle et 6-O-butanoyle dans des proportions variables selon les conditions opératoires. De plus, l'isomérisation de ce composé se poursuit de manière non contrôlable lorsque l'échantillon est mis en solution notamment en milieu aqueux. Cette isomérisation est conforme aux lois de la Chimie, lorsque dans un composé, un groupement hydroxyle primaire est stériquement proche d'un groupement ester lié à un site secondaire et que ce groupement hydroxyle a une orientation stérique favorable à l'attaque du groupement acyle voisin (P.Y. GOUETH et coll., J. Carbohydr. Chem. (1994), 13 (2), 249). Dès lors, il est impossible d'obtenir ce composé avec une pureté supérieure à 95%.

Un des buts de la présente invention est de préparer des esters associant les acides phénylacétique, 3-phénylpropionique, 4-phénylbutyrique own-butyrique à des oses ou des itols $Su(OH)_n$ choisis de sorte que leur structure ou celle de leurs dérivés évite le processus de transestérification interne responsable de l'isomérisation, tout en permettant une bonne biodisponibilité de l'acide correspondant.

Le but est atteint selon l'invention, par la synthèse de monoesters de formule générale :

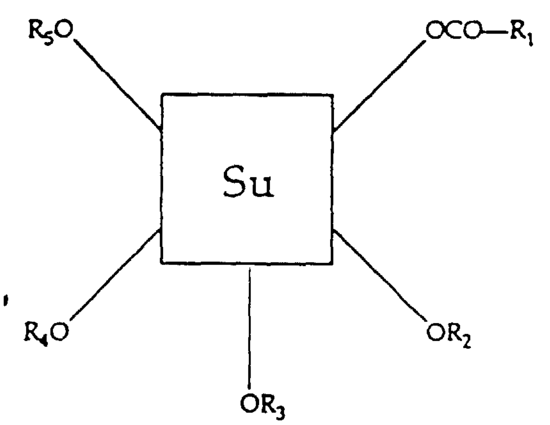

- dans laquelle l'ose ou l'itol $Su(OH)_n$, précurseur des composés conformes à l'invention, est de préférence le D-

mannose [Su(OH)$_5$] portant le groupement ester sur l'atome de carbone anomérique ou encore un pentitol [Su(OH)$_5$] comme, par exemple, le xylitol portant le groupement ester sur l'atome de carbone primaire C-1 ;

- dans laquelle $R_1$-CO est le groupement phénylacétyle, 3-phénylpropanoyle, 4-phénylbutanoyle, *n*-butanoyle ;
- dans laquelle $R_2$, $R_3$, $R_4$, $R_5$ peuvent être l'atome d'hydrogène ou des groupements comportant une chaîne hydrocarbonée, cycliques ou non, saturés ou non, ramifiés ou non, ou encore choisis de sorte que $R_2$ et $R_3$ et ou $R_4$ et $R_5$ appartiennent à un groupement acétal sous forme de dioxolane :

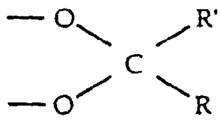

avec le groupement R-C-R' choisi de préférence parmi le phénylméthylène, le méthylène, le cyclohexylidène ou mieux l'isopropylidène ; et au moins deux des groupes $R_2$, $R_3$, $R_4$ et $R_5$ représentent un atome d'hydrogène

- et dans laquelle la transestérification interne en solution, est évitée parce que les composés ont, par rapport au groupement ester, des groupements hydroxyles libres éloignés et/ou mal orientés, et/ ou liés à un atome de carbone secondaire.

Lorsque, par exemple, la préparation des esters, conforme à la présente invention, est réalisée à partir des di-O-acétals du D-mannose ou du xylitol, la synthèse peut se dérouler, par exemple, selon l'étape *a* suivie éventuellement des étapes *b* et/ou *c*, comme illustré par le schéma 1 pour les composés du D-mannose de type A et B, et par le schéma 2 pour les composés du xylitol de type C.

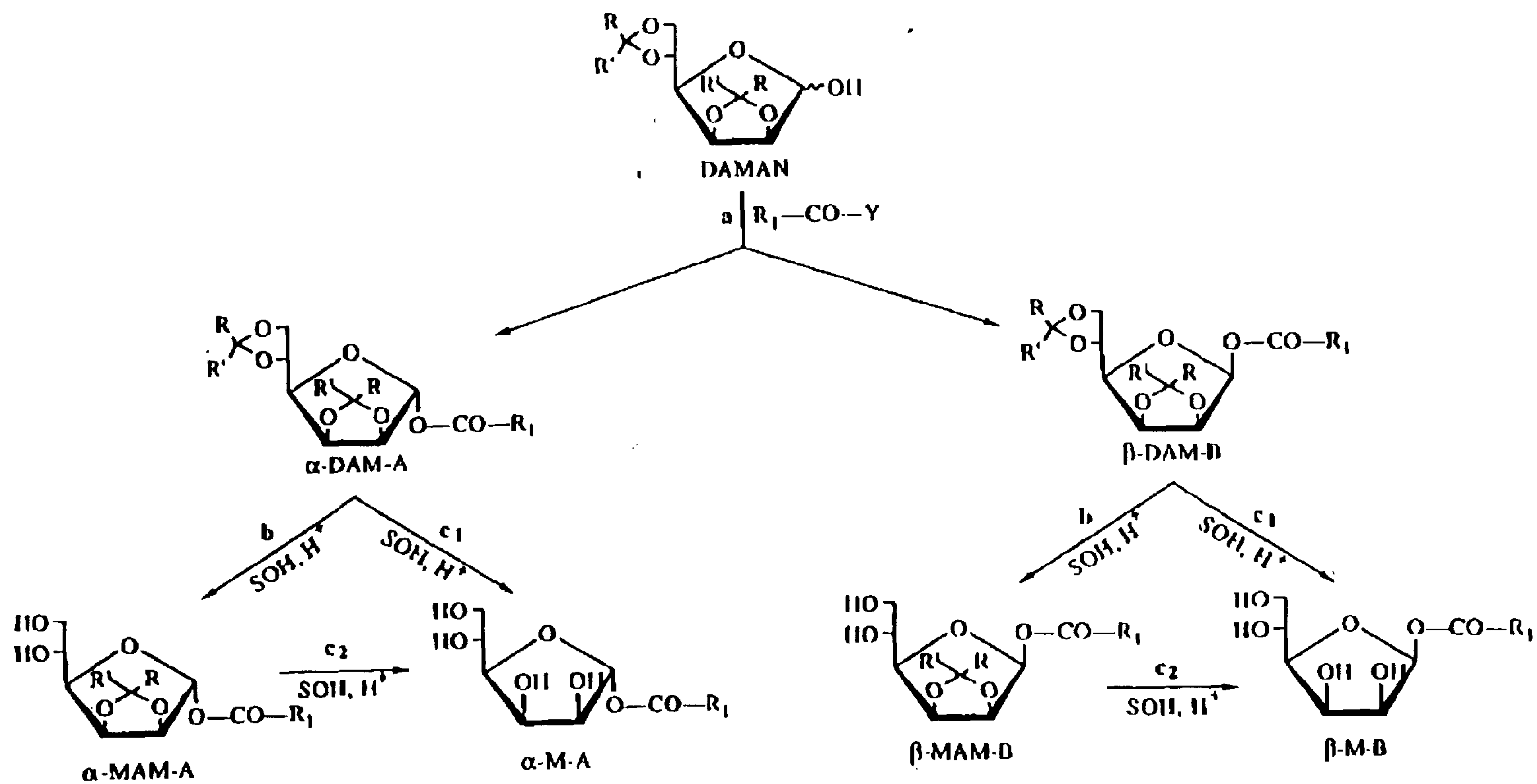


Y = Cl ; OH ; $R_1$—CO—O ; OR" (avec R" = $CH_3$ ; $C_2H_5$) ;

$R_1$ = Ph—$CH_2$ ; Ph—$(CH_2)_2$ ; Ph—$(CH_2)_3$ ; n-$C_3H_7$ ;

R—C—R' = Ph—CH ; $CH_2$ ; $C_6H_{10}$ ; $CH_3$—C—$CH_3$.

*Schéma I*

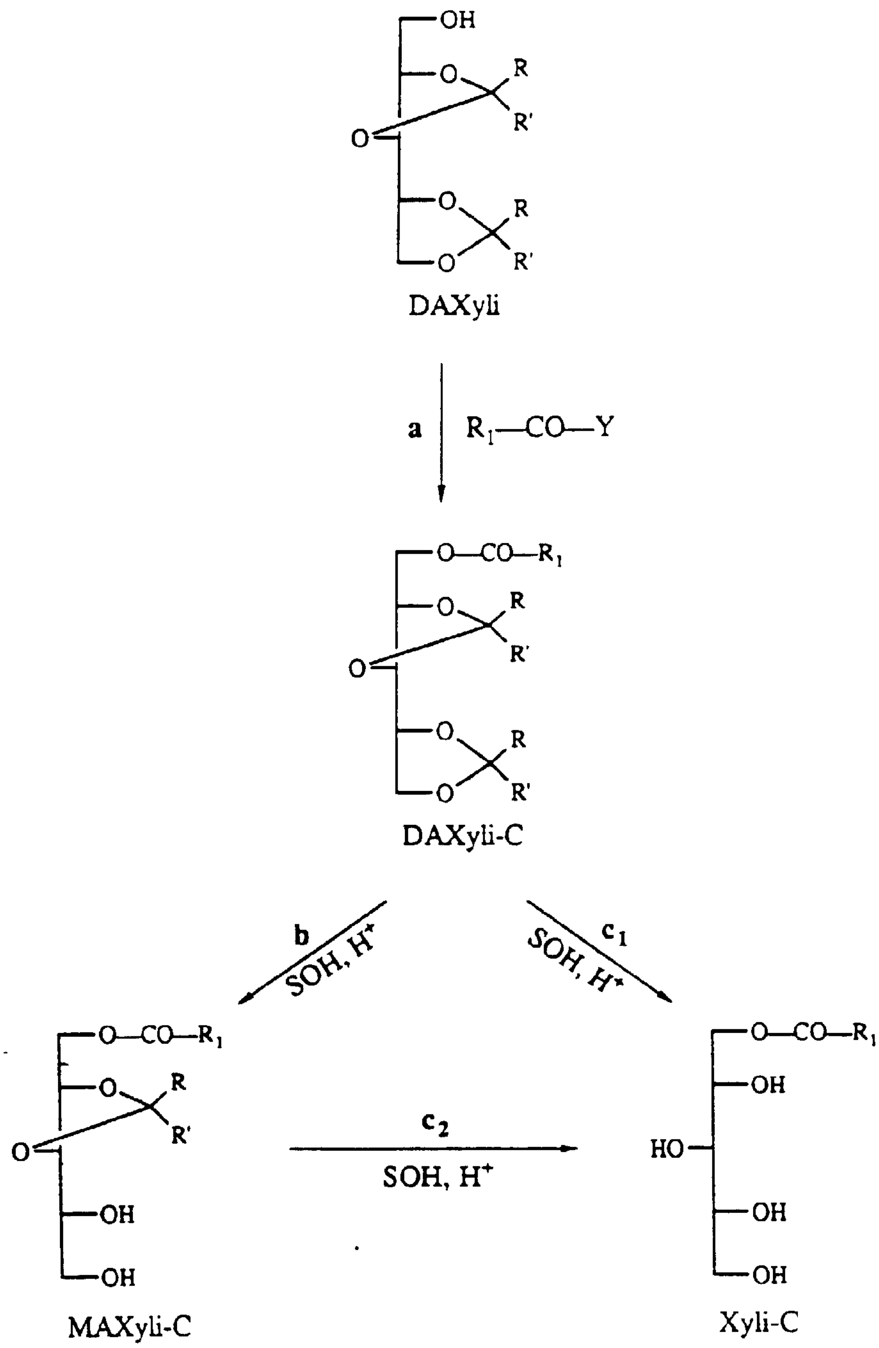


Y = Cl ; OH ; $R_1$—CO—O ; OR" (avec R" = $CH_3$ ; $C_2H_5$) ;

$R_1$ = Ph—$CH_2$ ; Ph—$(CH_2)_2$ ; Ph—$(CH_2)_3$ ; $n$-$C_3H_7$ ;

R—C—R' = Ph—CH ; $CH_2$ ; $C_6H_{10}$ ; $CH_3$—C—$CH_3$.

*Schéma 2*

*Etape a : Synthèse des esters α- et β-O-acyl-2,3:5,6-di-O-acétal-D-mannoside (α-DAM-A et β-DAM-B) et des esters 1-O-acyl-2,3:4,5-di-O-acétal-xylitol (DAXyli-C).*

Ces esters peuvent être préparés, par exemple, par addition d'un agent acylant $R_1$-CO-Y, au diacétal DAMannose et DAXylitol respectivement, en opérant éventuellement dans un solvant et en présence d'une base.

L'agent acylant peut être l'acide (Y = OH), l'anhydride d'acide (Y = O-CO-$R_1$), un ester (Y = OR", avec R" = $CH_3$ ou $C_2H_5$) ou mieux le chlorure d'acide (Y = Cl).

Le solvant peut être polaire ou apolaire, aliphatique ou aromatique, seul ou un mélange de ces solvants.

La base peut être une base faible minérale ou organique choisie, par exemple, parmi un carbonate alcalin, la pyridine ou mieux la triéthylamine.

L'acylation du diacétal DAMannose conduit au mélange d'esters anomères α-DAM-A et β-DAM-B. Ces esters sont séparés, par exemple, par chromatographie liquide du mélange.

*Etape b : Préparation des esters monoacétaliques α- et β-O-acyl-2,3-O-acétal-D-mannoside (α-MAM-A et β-MAM-B) et des esters 1-O-acyl-2,3-O-acétal-xylitol (MAXyli-C) par déprotection sélective des esters diacétaliques correspondants.*

La déprotection sélective d'un site acétalique selon l'étape *b* des schémas 1 et 2 est réalisée dans un solvant hydroxylé SOH, par acido-catalyse, soit en phase homogène, soit en phase hétérogène :

Le solvant hydroxylé peut être l'eau, un alcanol, un mélange eau-alcanol, un mélange alcanol-alcanol, ou encore eau ou alcanol associés à un cosolvant non hydroxylé. L'alcanol peut être choisi, par exemple, parmi le méthanol, l'éthanol, le propanol.

Le cosolvant peut être choisi, par exemple, parmi le dioxane, le tétrahydrofurane.

L'acido-catalyse en phase homogène peut être réalisée par l'addition au milieu, d'acides organique ou minéral. L'acide organique peut être choisi parmi les acides carboxyliques ou sulfoniques, comme par exemple, les acides formique, acétique, trifluoroacétique, paratoluènesulfonique. L'acide minéral peut être choisi, par exemple, parmi les acides sulfurique, chlorhydrique, nitrique, phosphorique. Les esters α-MAM-A, β-MAM-B et MAXyli-C obtenus, à l'état pur, après neutralisation du milieu par une base organique ou minérale, filtration, évaporation du solvant et purification par chromatographie liquide, par exemple.

L'acido-catalyse en phase hétérogène peut être réalisée par la présence, dans le milieu, d'une résine acide ou par percolation sur une colonne remplie de résine acide, de la solution d'ester dans le solvant hydroxylé (SOH) précédemment défini. Les esters α-MAM-A, β-MAM-B et MAXyli-C sont isolés à l'état pur après filtration, évaporation du solvant et purification par chromatographie liquide, par exemple.

Les dérivés du D-mannose de type α-MAM-A et β-MAM-B, ainsi que les dérivés du xylitol de type MAXyli-C qui, par rapport au groupement ester ont des groupements hydroxyles libres éloignés et/ou mal orientés, et/ou liés à un atome de carbone secondaire, ne peuvent donner lieu, en solution, à une transestérification interne.

*Etapes $c_1$ et $c_2$ : Préparation des esters α- et β-O-acyl-D-mannoside (α-M-A et β-M-B) et des esters 1-O-acyl-xylitol (Xyli-C) par déprotection des esters monoacétaliques ou diacétaliques correspondants.*

La déprotection de l'ester diacétalique selon l'étape $c_1$ ou de l'ester monoacétalique selon l'étape $c_2$ des schémas 1 et 2 peut être réalisée avec les mêmes solvants (SOH) et avec les mêmes acides que ceux définis pour l'étape *b* de la présente invention, en adaptant éventuellement la quantité d'acide, la température et la durée de la réaction.

Les dérivés du D-mannose de type α-M-A et de type β-M-B ainsi que les dérivés du xylitol de type Xyli-C qui, par rapport au groupement ester ont des groupements hydroxyles libres éloignés, et/ou mal orientés, et/ou liés à un atome de carbone secondaire, ne peuvent donner lieu, en solution, à une transestérification interne.

Un autre but de l'invention est l'utilisation comme médicaments, des esters associant les acides phénylacétique, 3-phénylpropionique, 4-phénylbutyrique ou *n*-butyrique au D-mannose, au xylitol et à leurs dérivés, de formule générale :

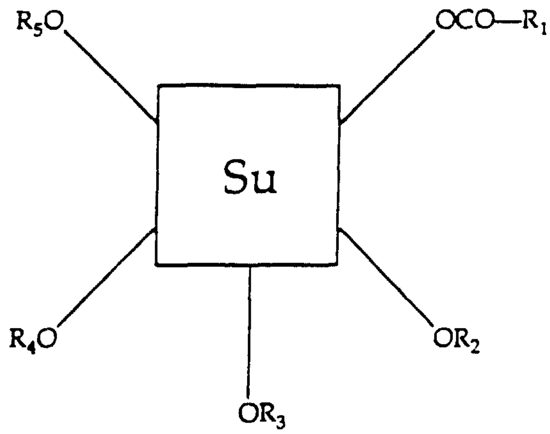

L'utilisation vise notamment, comme avec les acides et les sels correspondants, et sans que ce soit limitatif, les hémoglobinopathies comme, par exemple, les anémies, la β-thalassémie, la drépanocytose ou encore les tumeurs prémalignes et malignes comme, par exemple, le cancer du sein, du colon, les leucémies aiguës et chroniques.
Les esters décrits dans la présente invention se distinguent aussi des sels et des esters correspondants par une vie plasmatique beaucoup plus longue, qui entraîne une biodisponibilité très supérieure, permettant d'envisager chez l'Homme des traitements à long terme.
A titre d'exemples et sans que cela soit considéré comme limitatif, on décrit ci-après, la préparation d'esters du D-mannose, du xylitol et de leurs dérivés, conformes à la présente invention.

***Exemple n° 1- Préparation des α- et β-O-acyl-2,3,5,6-di-O-isopropylidène-D-mannofuranosides (α-DAM-A et β-DAM-B).***

*a) Estérification du diacétonemannose par le chlorure de n-butanoyle.*

Dans un réacteur de 3L, thermostaté à 60°C, contenant 2,6 L de toluène, on dissout sous agitation 260 g (1 mol) de diacétonemannose (2,3:5,6-di-O-isopropylidéne-D-mannose). La solution est portée à ébullition sous pression réduite à 60°C pour éliminer, par distillation, les traces d'eau éventuelles sous forme d'azéotrope eau-toluène. Après avoir récupéré 100 mL de distillat, le contrôle par la méthode de Karl-Fischer montre que la solution dans le réacteur est rigoureusement anhydre. On ramène alors le milieu à la pression atmosphérique, et à température ambiante, on verse 106 g (1,05 mol) de triéthylamine (TEA) puis, goutte à goutte et sous agitation, 106,5 g (1 mol) de chlorure de *n*-butanoyle (durée de l'addition : 10 minutes). Un contrôle effectué par CPV (colonne OV17) indique la disparition totale du diacétonemannose après 60 minutes de réaction.

La solution toluènique est filtrée afin d'éliminer le chlorhydrate de TEA. Le filtrat évaporé sous pression réduite donne 332 g de produit brut sirupeux. La composition déterminée par CPV (colonne OV 17) est de 90% d'anomère α et de 10% d'anomère β.

*b) Séparation des butyrates anomères α-DAM-B et β-DAM-B.*

Aux 332 g de produit brut précédent, on ajoute 500 mL d'acétone et 300 g de silice neutre 60Å, 35-40 mesh. Ce mélange est évaporé sous pression réduite. Le magma est transféré sur une colonne (diamètre 40 mm) contenant 700 g de silice. L'élution est réalisée par le mélange hexane-acétone avec un gradient croissant en acétone.

On isole successivement :

- après passage de 10 L du mélange hexane-acétone (97:3, v/v), 290 g d'anomère *a* pur, liquide à la température ambiante ;
  $[\alpha]_D^{12}$ = +43,5° (c = 1,1; $CHCl_3$) ; les spectres RMN $^1H$ et $^{13}C$ (tableau 1) sont conformes à la structure de l'anomère α;
- après passage de 1 L du mélange hexane-acétone (96:4, v/v) 1,5 g de mélange α, β;
- après passage de 4 L du mélange hexane-acétone (90:10, v/v), 28 g d'anomère β pur, liquide à la température ambiante ;
  $[\alpha]_D^{20}$ = +23,2° (c = 1,2 ; $CHCl_3$) ; les spectres RMN $^1H$ et $^{13}C$ (tableau 2) sont conformes à la structure

de l'anomère β.

La masse totale des esters récupérés est de 319,5 g, soit un rendement de 97% par rapport au diacétonemannose. Le rendement final en butyrates de diacétonemannose anomères purifiés est donc de 88% en ester α et de 9% en ester β.

c) *Préparation des α- et β-O-acyl-2,3:5,6-di-O-isopropylidène-D-mannosides (α-DAM-A et β-DAM-B), avec acyle = Ph-$(CH_2)_n$-O et n=1;2;3.*

Ces esters sont préparés dans les mêmes conditions que les *n*-butyrates correspondants (cf. α) mais avec des quantités dix fois plus faibles. Le tableau ci-après donne la durée de la réaction, la distribution α/β des anomères, la composition de l'éluant de chromatographie pour isoler l'anomère α, le rendement et les constantes physiques de ce dernier.

| | | | α-DAM-A | | | | |
|---|---|---|---|---|---|---|---|
| n | durée (h) | α/β | % Hex.-Ac. (v/v) | Rdt isolé (%) | $[\alpha]_D$ dans $CHCl_3$ (T °C:c = %) | F (°C) | Tableau RMN |
| 1 | 2,5 | 81/19 | 95:5 | 75 | +18,2° (25 ; 1,6) | 79-82 | 3 |
| 2 | 2,5 | 83/17 | 95:5 | 75 | +34,6° (26; 1,6) | 105,4-105,7 | 4 |
| 3 | 4,0 | 86/14 | 94:6 | 76 | +33,3° (27; 1,1) | liquide | 5 |

Les spectres RMN $^1H$ et $^{13}C$ sont conformes à la structure des produits attendus.

*Exemple n° 2- Préparation des α-O-acyl-2,3-O-isopropylidène-D-mannofuranosides (α-MAM-A).*

a) *Déprotection sélective acidocatalysée du butyrate α-DAM-A.*

*a.1) Conditions expérimentales et vitesse de déprotection sur résines acides.*

Dans un ballon thermostaté à 51°C, on met sous agitation, dans 135 mL d'éthanol à 96°, 20 g de résine acide Amberlite 15H+ wet et 15 g d'ester α. Le suivi cinétique par dosage HPLC (colonne RP 18) de prélèvements espacés montre que la constante de vitesse de déprotection selon la loi de pseudo-premier ordre est $k = 1{,}10.10^{-2}$ $min^{-1}$, ce qui correspond a un taux d'avancement de 93% après 4 heures de réaction. L'analyse HPLC (colonne RP 18) du prélèvement effectué après 4 heures montre la présence de 2 signaux d'intensités relatives 93/7. Le plus intense correspond à un produit plus polaire, identifié par les analyses décrites ci-après en 2-*b*, comme étant le O-*n*-butanoyl-2,3-O-isopropylidène-α-D-mannoside.

Dans ces conditions expérimentales il y a déprotection hautement sélective du groupement 5,6-O-isopropylidène.

a.2) *Déprotection quantitative du butyrate α-DAM-A* sur résines *acides.*

On procède comme en *a.1,* avec 2,25 L d'éthanol à 96°, 187,5 g de résine acide et 250 g d'ester α-DAM-A. Après 4 heures de réaction, on filtre le mélange, on rince les résines avec 2 fois 200 mL d'éthanol à 96°. L'évaporation des solutions alcooliques rassemblées permet de récupérer 220 g de résidu solide.

*a.3) Déprotection sélective acidocatalysée du butyrate α-DAM-A en milieu homogène.*

La déprotection de l'ester a été également réalisée en solution homogène dans un ballon thermostaté à 30°C, contenant 100 mL d'éthanol à 96°, ou 100 mL de solvant dioxane-eau (80:20, v/v), avec $H_2SO_4$ 0,2 N et 15 g de butyrate α-DAM-A.

Le taux de déprotection dépasse 90%, aprés 4 heures de réaction. L'extraction du produit implique la neutralisation par une solution aqueuse de lessive de soude. Pour éviter la désestérification du produit, l'ajout de la base doit être fait à basse température (0 à 10°C) et arrêté dès que le pH du milieu atteint 7 unités. Le produit brut est isolé après séparation du sulfate de sodium par filtration et évaporation du solvant sous pression réduite.

*b) Purification du butyrate α-MAM-A.*

Aux 220 g de résidu solide précédent, on ajoute 500 mL de toluène et 250 g de silice neutre 60Å, 35-40 mesh. Ce mélange est évaporé sous pression réduite. Le magma est transféré sur une colonne (diamètre 40 mm) contenant 750 g de silice. L'élution est réalisée par le mélange hexane-acétone avec un gradient croissant en acétone.

On isole successivement :

- après passage de 4,5 L de mélange hexane-acétone (85:15, v/v), 15 g d'ester α-DAM-A ;
- après passage de 6 L de mélange hexane-acétone (70:30, v/v), 190 g de O-*n*-butanoyl-2,3-O-isopropylidène-α-D-mannoside-α-MAM-A;
  $[\alpha]_D^{22}$ = +54,6° (c = 1,2; $CHCl_3$) ;
  F = 79°C ; les spectres RMN $^1H$ et $^{13}C$ (tableau 6) sont conformes à la structure du produit attendu ;
- après passage de 1 L du mélange hexane-acétone (50:50, v/v), puis de 2 L d'acétone pure, 6 g de mélange contenant majoritairement le O-n-butanoyl-α-D-mannoside, puis 3 g de O-n-butanoyl-α-Dmannoside, pur, d'après l'analyse HPLC.

*c) Préparations des esters O-acyl-2,3-O-isopropylidène-α-D-mannofuranosides (α-MAM-A), avec acyle = Ph-$(CH_2)_n$-CO et n = 1 ; 2 ; 3.*

Ces esters sont préparés dans les mêmes conditions que les *n*-butyrates correspondants (cf. *a*.2) mais avec des quantités dix fois plus faibles. Le tableau ci-après donne la durée de la réaction, la composition de l'éluant de chromatographie, le rendement et les constantes physiques.

| n | durée (h) | Hex.-Ac. (v/v) | Rdt isolé (%) | $[\alpha]_D$ dans $CHCl_3$ (T °C ; c = %) | F (°C) | Tableau RMN |
|---|---|---|---|---|---|---|
| 1 | 4,0 | 75:25 | 75 | +32,2° (25 ; 1,6) | liquide | 7 |
| 2 | 4,5 | 70:30 | 77 | +34,6° (26 ; 1,6) | 30,5-33,6 | 8 |
| 3 | 3,5 | 75:25 | 77 | +33,3° (27; 1,1) | 70,2-72,6 | 9 |

Les spectres RMN $^1H$ et $^{13}C$ sont conformes à la nature des produits attendus.

***Exemple n° 3- Préparation des 1-O-acyl-2,3:4,5-di-O-isopropylidène-xylitol (DAXyli-C).***

*a) Estérification du diacétonexylitol par le chlorure de n-butanoyle.*

Dans un réacteur de 3L, thermostaté à 60°C, contenant 800 mL de toluène, on dissout sous agitation 100 g (0,43 mol) de diacétonexylitol (2,3:4,5-di-O-isopropylidène-xylitol). La solution est portée à ébullition sous pression réduite à 60°C pour éliminer, par distillation, les traces d'eau éventuelles sous forme d'azéotrope eau-toluène. Après avoir récupéré 40 mL de distillat, le contrôle par la méthode de Karl-Fischer montre que la solution dans le réacteur est rigoureusement anhydre. On ramène alors le milieu à la pression atmosphérique, et à température ambiante, on verse 47,1 g (0,42 mol) de triéthylamine (TEA) puis, goutte à goutte et sous agitation, 45,9 g (0,43 mol) de chlorure de butanoyle (durée de l'addition 7 minutes). Un contrôle effectué par CPV (colonne OV17) indique la disparition totale du diacétonexylitol après 4 heures de réaction.

La solution toluènique est filtrée afin d'éliminer le chlorhydrate de TEA. Le filtrat évaporé sous pression réduite donne 131 g de produit brut sirupeux.

*b) Purification du butyrate DAXyli-C.*

Aux 131 g de produit brut précédent, on ajoute 250 mL d'acétone et 100 g de silice neutre 60Å, 35-40 mesh. Ce mélange est évaporé sous pression réduite. Le magma est transféré sur une colonne (diamètre 40 mm) contenant 400 g de silice. L'élution est réalisée par le mélange hexane-acétone avec un gradient croissant en acétone.

On isole, après passage du mélange hexane-acétone (98:2, v/v), 108,7 g d'ester pur, soit un rendement de 84% par rapport au diacétonexylitol ;

$[\alpha]_D^{23}$ = -0,3° (c = 1,7 ; $CHCl_3$) ; les spectres RMN $^1H$ et $^{13}C$ (tableau 10) sont conformes à la structure de l'anomère α.

*c) Préparation des esters 1-O-acyl-2,3:4,5-di-O-isopropylidène-xylitol, avec acyle = Ph-$(CH_2)_n$-O et n = 1 ; 2 ; 3.*

Ces esters sont préparés sont préparés dans les mêmes conditions que les n-butyrates correspondants (cf. *a*), mais avec des quantités dix fois plus faibles. Ces composés sont liquides à la température ambiante. Le tableau ci-après donne la durée de la réaction, la composition de l'éluant de chromatographie, les rendements et les constantes physiques.

| n | durée (h) | Hex.-Ac. (v/v) | Rdt isolé (%) | $[\alpha]_D$ dans $CHCl_3$ (T °C ; c = %) | Tableau RMN |
|---|---|---|---|---|---|
| 1 | 5,0 | 90:10 | 86 | -1,6° (29 ; 1,0) | 11 |
| 2 | 3,0 | 95:5 | 91 | -1,1° (29 ; 1,0) | 12 |
| 3 | 4,0 | 90:10 | 89 | -1,7° (29 ; 1,1) | 13 |

Les spectres RMN $^1H$ et $^{13}C$ sont conformes à la structure des produits attendus.

***Exemple n° 4- Préparation des 1-O-acyl-2,3-O-isopropylidène-xylitol (MAXyli-C) et des 1-O-acyl-xylitol (Xyli-C).***

*a) Déprotections sélective et totale acido-catalysées du butyrate de DAXyli-C.*

Dans un ballon thermostaté à 52°C, on met sous agitation, dans 800 mL d'éthanol à 96°, 200 g de résine acide Amberlyst $15H^+$ wet et 100 g d'ester. Le suivi cinétique par dosage HPLC (colonne RP 18) de prélèvements espacés montre que l'on a un taux d'avancement de 98% après 4 heures de réaction. L'analyse HPLC du prélèvement montre la présence de 2 signaux d'intensités relatives 7/3. Le plus intense correspond à un produit plus polaire, identifié par les analyses décrites ci-après en *4-b,* comme étant le 1-O-*n*-butanoyl-xylitol. Après 4 heures de réaction, on filtre le mélange, on rince les résines avec 2 fois 200 mL d'éthanol à 96°. L'évaporation des solutions alcooliques rassemblées permet de récupérer 79 g de liquide sirupeux.

*b) Séparation des butyrates MAXyli-C et Xyli-C.*

Aux 79 g de produit brut précédent, on ajoute 150 mL d'éthanol à 96° et 80 g de silice neutre 60Å, 35-40 mesh. Ce mélange est évaporé sous pression réduite. Le magma est transféré sur une colonne (diamètre 30 mm) contenant 320 g de silice. L'élution est réalisée par le mélange acétone-hexane industriel avec un gradient croissant en acétone.

On isole successivement :

- après passage de 7 L du mélange hexane-acétone (75:25, v/v), 25 g d'ester MAXyli-C pur, liquide à la température ambiante ;
  $[\alpha]_D^{26}$ = +0,3° (c = 1,2; $CHCl_3$) ; les spectres RMN $^1H$ et $^{13}C$ (tableau 14) sont conformes à la structure du produit attendu ;
- après passage du mélange hexane-acétone (20:80, v/v), 46,8 g d'ester Xyli-C pur, liquide à la température ambiante;
  $[\alpha]_D^{26}$ = +1,3° (1,1 ; $CHCl_3$); les spectres RMN $^1H$ et $^{13}C$ (tableau 15) sont conformes à la structure du produit attendu.

*c) Préparation des esters 1-O-acyl-2,3-O-isopropylidène-xylitol (MAXyli-C) et 1-O-acyl-xylitol (Xyli-C), avec acyle = Ph-$(CH_2)_n$-O et n=1;2;3.*

Ces esters sont préparés dans les mêmes conditions que les *n*-butyrates correspondants (cf. *a*), mais avec des quantités dix fois plus faibles. Le tableau ci-après donne la durée de la réaction, la distribution des esters MAXyli-C et Xyli-C, la composition de l'éluant pour isoler chacun d'eux, le rendement et les constantes physiques de ces composés.

| n | durée (h) | Produit | Hex.-Ac. (v/v) | Rdt isolé (%) | $[\alpha]_D$ dans MeOH (T °C ; c = %) | F (°C) | Tableau RMN |
|---|---|---|---|---|---|---|---|
| 1 | 2,5 | MAXyli-C | 88:22 | 32 | -4,1° (30 ; 1,2) | liquide | 16 |
| | | Xyli-C | 40:60 | 56 | -2,0° (30 ; 1,0) | 66,8-69,3 | 17 |
| 2 | 3,0 | MAXyli-C | 75:25 | 36 | -5,5° (31 ; 1,0) | liquide | 18 |
| | | Xvli-C | 35:65 | 60 | -1,7° (30 ; 1,1) | liquide | 19 |
| 3 | 2,5 | MAXyli-C | 75:25 | 36 | -3,2° (31 ; 1,8) | liquide | 20 |
| | | Xyli-C | 25:75 | 62 | -3,0° (30 ; 1,0) | 39,5-42,8 | 21 |

Les spectres RMN $^1H$ et $^{13}C$ sont conformes à la structure des produits attendus.

***Exemple n° 5- Stabilité chimique des esters en milieu aqueux.***

Les esters α-O-acyl-2,3-O-isopropylidène-mannoside décrits dans l'exemple n°2 ainsi que les esters 1-O-acyl-2,3-O-isopropylidène-xylitol et 1-O-acyl-xylitol décrits dans l'exemple n°4 ont été soumis à un contrôle prolongé de stabilité en solution dans les conditions ci-après :

1. dans l'eau distillée (pH = 6,5) à 4°C et à 25°C ;
2. dans le mélange eau-éthanol 50:50 (v/v) à 25°C ;
3. dans l'eau distillée avec $CO_3HNa$ 0,2 mol.$L^{-1}$ (pH = 8,1) à 25°C.

*Résultats*

- Dans les conditions 1. et 2., le contrôle éffectué par HPLC (colonne RP 18, éluant eau-acétone) montre que la perte d'ester par hydrolyse est inférieure à 1% après un mois en solution. En outre l'évaporation après un mois, suivie d'une chromatographie liquide permet de récupérer l'ester pur, d'après ses constantes physiques et spectroscopiques, dont la masse représente 98% de la masse de l'ester initialement dissous.
- Dans les conditions 3., les contrôles éffectués par HPLC comme pour 1. et 2. montrent une désestérification moins lente des esters que dans les conditions précédentes à cause de la basicité du milieu (pH = 8,1 contre 6,5 dans les conditions 1.). Cette désestérification a lieu à une vitesse voisine de celle observée pour le 3-O-*n*-butanoyl-1,2-O-isopropylidène-α-D-glucofuranose dans les mêmes conditions (Goueth et coll., J. Carbohydr. Chem. (1994), 13 (2), 249), soit 20% en 95 heures contre 15 à 25% pour les esters étudiés. Cependant, pour chacun des esters décrits ici, la fraction d'ester récupérée après 95 heures a les mêmes constantes physiques et spectroscopiques que le produit initialement dissous, tandis que pour le 3-O-*n*-butanoyl-1,2-O-isopropylidène-α-D-glucofuranose, la fraction non désestérifiée se compose de 6% de l'ester initial, 90% de l'isomère 6-O-*n*-butanoyle et 4% de l'isomère 5-O-*n*-butanoyle.

***Exemple n° 6- Mise en évidence des propriétés antiprolifératives et de maturation cellulaire des esters butyriques α-MAM-A, MAXyli-C et Xyli-C ($R_1$=n-$C_3H_7$) sur des lignées responsables de la leucémie myéloïde aigüe.***

Les essais sont menés sur des lignées cellulaires stabilisées (HL 60 et autres) en l'absence de facteurs de croissance. Les concentrations en dérivés butyriques varient de 0,5 à 1 mM.

Les paramètres suivants ont été évaluées par balayage "FASC Scan" et cytométrie de flux :

- incorporation de la thymidine tritiée (exploration du métabolisme cellulaire) ;
- incorporation d'iodure de propylium (détermination de la phase du cycle cellulaire) ;
- évaluation du taux de cellules en phase d'apoptose (mort cellulaire programée) ;
- évaluation de la maturation cellulaire (dosage des antigénes de surface par des anticorps monoclonaux spécifiques).

Les propriétés antiprolifératives sont démontrées par les données du tableau ci-après sur le pourcentage d'inhibition de croissance cellulaire et sur le pourcentage de cellules en apoptose.

| Composés butyriques ($R_1$=*n*-$C_3H_7$) | % d'inhibition de croissance 1IL(λ) (n jours) | % d'apoptose HL 60 et autres (3 jours) |
|---|---|---|
| α-MAM-A | 40(2 j.)<br>80 (5 j) | 13 à 84 selon la lignée |
| MAXyli-C | 80 (3 j.)<br>90 (4 j.) | 27 à 96 selon la lihnée |
| Xyli-C | 70 (5 j.) | 10 à 35 selon la lignée |

La maturation des cellules myéloïdes est évaluée par l'expression des antigènes spécifiques CD 15, CD 11a et CD 11b. Les trois produits ci-dessus , à la concentration 1mM ne provoquent qu'une augmentation marginale des

antigènes spécifiques de maturation.

***Exemple n° 7- Mise en évidence de l'effet des esters butyriques α-MAM-A et Xyli-C ($R_1$=n-$C_3H_7$) sur la synthèse d'hémoglobine foetale.***

Les essais ont été menés sur des lignées cellulaires érythroleucémiques en présence de sérum humain normal.

Les paramètres suivants ont été évalués :

- taux dc chaîne γ de l'hémoglobine (dosage immunologique par double réaction antigène anticorps, et par dosage immunocytochimique en présence de phosphatase alcaline, de phosphatase antialcaline A.P.A.A.P.) ;
- taux d'$ARN_m$ des chaînes γ (Northern Blot Analysis).

Il est observé que les esters α-MAM-A et Xyli-C au concentrations respectives de 0,70 mM et 0,35 mM augmentent le taux de chaîne γ de l'hémoglobine.

En présence d'A.P.A.A.P., ce taux passe de 10% sans ester à 22% avec α-MAM-A et à 17% avec Xyli-C. Le test de détection des $ARN_m$ des chaînes γ en présence des esters ci-dessus montre de manière très significative l'augmentation des $ARN_m$.

Tableau 1 : Spectres RMN $^{1}H$ et $^{13}C$ du O-*n*-butanoyl-2,3:5,6-di-O-isopropylidène-α-D-mannoside.

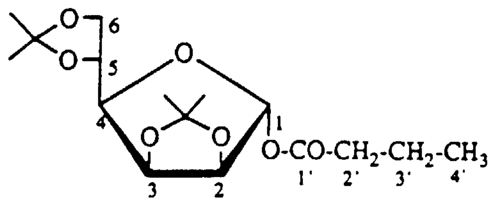

| C | δ (ppm) |
|---|---|
| $C_1$ | 99,4 |
| $C_2$ | 83,9 |
| $C_3$ | 78,2 |
| $C_4$ | 81,1 |
| $C_5$ | 71,8 |
| $C_6$ | 65,7 |
| $C_{iso}$ | 112,0 |
| $C_{iso}$ | 108,1 |
| $CH_3$ | 25,8 |
| $CH_3$ | 24,8 |
| $CH_3$ | 24,0 |
| $CH_3$ | 23,5 |
| $C_{1'}$ | 170,7 |
| $C_{2'}$ | 34,9 |
| $C_{3'}$ | 17,1 |
| $C_{4'}$ | 12,4 |

| H | δ (ppm) | | J (Hz) |
|---|---|---|---|
| $H_1$ | 5,94 | s | $J_{1-2} = 0$ |
| $H_2$ | 4,50 | d | $J_{2-3} = 5,8$ |
| $H_3$ | 4,67 | dd | $J_{3-4} = 3,7$ |
| $H_4$ | 3,82 | m | $J_{4-5} = 4,4$ |
| $H_5$ | 4,20 | m | $J_{5-6a} = 6,2$ |
| $H_{6a}$ | 3,90 | dd | $J_{6a-6b} = 8,7$ |
| $H_{6b}$ | 3,82 | m | $J_{5-6b} = 7,6$ |
| $CH_{3\ iso}$ | 1,29 | s | -- |
| $CH_{3\ iso}$ | 1,26 | s | -- |
| $CH_{3\ iso}$ | 1,18 | s | -- |
| $CH_{3\ iso}$ | 1,15 | s | -- |
| $H_{2'}$ | 2,10 | t | $J_{2'-3'} = 7,2$ |
| $H_{3'}$ | 1,46 | q | $J_{3'-4'} = 7,4$ |
| $H_{4'}$ | 0,76 | t | -- |

(Solvant : $CDCl_3$ ; déplacement chimique δ par rapport au signal du TMS).

Tableau 2 : Spectres RMN $^1H$ et $^{13}C$ du O-*n*-butanoyl-2,3:5,6-di-O-isopropylidène-β-D-mannoside.

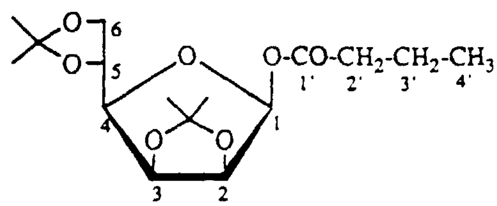

| C | δ (ppm) |
|---|---|
| $C_1$ | 95,4 |
| $C_2$ | 77,8 |
| $C_3$ | 78,4 |
| $C_4$ | 77,4 |
| $C_5$ | 72,1 |
| $C_6$ | 65,7 |
| $C_{iso}$ | 113,2 |
| $C_{iso}$ | 108,3 |
| $CH_3$ | 25,9 |
| $CH_3$ | 24,6 |
| $CH_3$ | 24,3 |
| $CH_3$ | 24,2 |
| $C_{1'}$ | 170,6 |
| $C_{2'}$ | 34,9 |
| $C_{3'}$ | 17,0 |
| $C_{4'}$ | 12,5 |

| H | δ (ppm) | | J (Hz) |
|---|---|---|---|
| $H_1$ | 5,66 | d | $J_{1-2}$ = 2,9 |
| $H_2$ | 4,63 | m | $J_{2-3}$= n.d. |
| $H_3$ | 4,63 | m | $J_{3-4}$ = 2,8 |
| $H_4$ | 3,62 | dd | $J_{4-5}$ = 7,8 |
| $H_5$ | 4,24 | m | $J_{5-6a}$ = n.d. |
| $H_{6a}$ | 3,87 | | $J_{6a-6b}$ = n.d. |
| $H_{6b}$ | Syst. | AA'X | $J_{5-6b}$= n.d. |
| $CH_{3\ iso}$ | 1,32 | s | -- |
| $CH_{3\ iso}$ | 1,26 | s | -- |
| $CH_{3\ iso}$ | 1,17 | s | -- |
| $CH_{3\ iso}$ | 1,17 | s | -- |
| $H_{2'}$ | 2,16 | t | $J_{2'-3'}$= 7,4 |
| $H_{3'}$ | 1,48 | q | $J_{3'-4'}$= 7,4 |
| $H_{4'}$ | 0,76 | t | -- |

(Solvant : $CDCl_3$ ; déplacement chimique δ par rapport au signal du TMS).

Tableau 3 : Spectres RMN $^{1}H$ et $^{13}C$ du O-phénylacétyl-2,3:5,6-di-O-isopropylidène-α-D-mannoside.

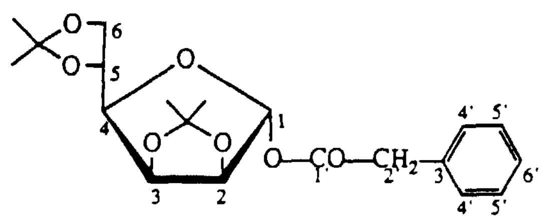

| C | δ (ppm) | H | δ (ppm) | | J (Hz) |
|---|---|---|---|---|---|
| $C_1$ | 100,9 | $H_1$ | 6,11 | s | $J_{1-2} = 0$ |
| $C_2$ | 84,9 | $H_2$ | 4,63 | d | $J_{2-3} = 5,8$ |
| $C_3$ | 79,3 | $H_3$ | 4,78 | dd | $J_{3-4} = 3,5$ |
| $C_4$ | 82,4 | $H_4$ | 3,88 | m | $J_{4-5} = 8,0$ |
| $C_5$ | 72,7 | $H_5$ | 4,35 | m | $J_{5-6a} = 6,2$ |
| $C_6$ | 66,6 | $H_{6a}$ | 4,05 | dd | $J_{6a-6b} = 8,8$ |
| $C_{iso}$ | 113,2 | $H_{6b}$ | 3,93 | m | $J_{5-6b} = 4,4$ |
| $C_{iso}$ | 109,2 | $CH_{3\ iso}$ | 1,43 | s | -- |
| $CH_3$ | 26,8 | $CH_{3\ iso}$ | 1,40 | s | -- |
| $CH_3$ | 25,9 | $CH_{3\ iso}$ | 1,34 | s | -- |
| $CH_3$ | 25,1 | $CH_{3\ iso}$ | 1,29 | s | -- |
| $CH_3$ | 24,6 | $H_{2'}$ | 2,13 | s | -- |
| $C_{1'}$ | 169,7 | $H_{4'}$ | 7,22 | | -- |
| $C_{2'}$ | 41,3 | $H_{5'}$ | à | m | -- |
| $C_{3'}$ | 133,4 | $H_{6'}$ | 7,33 | | -- |
| $C_{4'}$ | 129,0 | | | | |
| $C_{5'}$ | 128,6 | | | | |
| $C_{6'}$ | 127,2 | | | | |

(Solvant : $CDCl_3$ ; déplacement chimique δ par rapport au signal du TMS).

Tableau 4 : Spectres RMN $^1$H et $^{13}$C du O-3'-phénylpropanoyl-2,3:5,6-di-O-isopropylidène-α-D-mannoside.

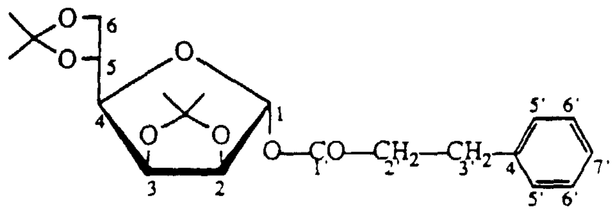

| C | δ (ppm) |
|---|---|
| $C_1$ | 100,9 |
| $C_2$ | 85,0 |
| $C_3$ | 79,2 |
| $C_4$ | 82,3 |
| $C_5$ | 72,8 |
| $C_6$ | 66,2 |
| $C_{iso}$ | 113,1 |
| $C_{iso}$ | 109,2 |
| $CH_3$ | 26,8 |
| $CH_3$ | 26,6 |
| $CH_3$ | 25,1 |
| $CH_3$ | 24,9 |
| $C_{1'}$ | 171,1 |
| $C_{2'}$ | 35,9 |
| $C_{3'}$ | 30,7 |
| $C_{4'}$ | 140,0 |
| $C_{5'}$ | 128,4 |
| $C_{6'}$ | 128,1 |
| $C_7$ | 126,3 |

| H | δ (ppm) | | J (Hz) |
|---|---|---|---|
| $H_1$ | 6,08 | s | $J_{1-2} = 0$ |
| $H_2$ | 4,54 | d | $J_{2-3} = 5,8$ |
| $H_3$ | 4,74 | dd | $J_{3-4} = 3,6$ |
| $H_4$ | 3,91 | m | $J_{4-5} = 7,8$ |
| $H_5$ | 4,35 | m | $J_{5-6a} = 6,2$ |
| $H_{6a}$ | 4,06 | dd | $J_{6a-6b} = 8,8$ |
| $H_{6b}$ | 3,97 | m | $J_{5-6b} = 4,5$ |
| $CH_3$ iso | 1,44 | s | -- |
| $CH_3$ iso | 1,42 | s | -- |
| $CH_3$ iso | 1,34 | s | -- |
| $CH_3$ iso | 1,29 | s | -- |
| $H_{2'}$ | 2,92 | t | $J_{2'-3'} = 7,4$ |
| $H_{3'}$ | 2,62 | t | -- |
| $H_{5'}$ | 7,27 | | -- |
| $H_{6'}$ | à | m | -- |
| $H_{7'}$ | 7,18 | | -- |

(Solvant : $CDCl_3$ ; déplacement chimique δ par rapport au signal du TMS).

Tableau 5 : Spectres RMN $^1H$ et $^{13}C$ du O-4'-phénylbutanoyl-2,3:5,6-di-O-isopropylidène-α-D-mannoside.

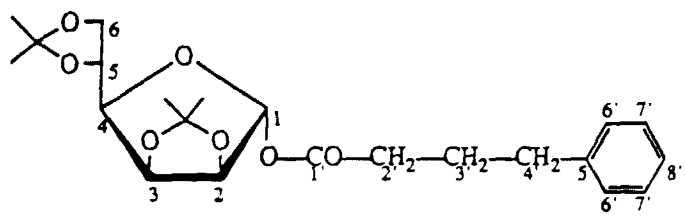

| C | δ (ppm) |
|---|---|
| $C_1$ | 100,7 |
| $C_2$ | 85,0 |
| $C_3$ | 79,3 |
| $C_4$ | 82,3 |
| $C_5$ | 72,8 |
| $C_6$ | 66,7 |
| $C_{iso}$ | 113,2 |
| $C_{iso}$ | 109,2 |
| $CH_3$ | 26,8 |
| $CH_3$ | 26,0 |
| $CH_3$ | 25,1 |
| $CH_3$ | 24,6 |
| $C_{1'}$ | 171,6 |
| $C_{2'}$ | 34,9 |
| $C_{3'}$ | 33,4 |
| $C_{4'}$ | 25,9 |
| $C_{5'}$ | 141,0 |
| $C_{6'}$ | 128,3 |
| $C_{7'}$ | 128,3 |
| $C_{8'}$ | 126,0 |

| H | δ (ppm) | | J (Hz) |
|---|---|---|---|
| $H_1$ | 6,11 | s | $J_{1-2} = 0$ |
| $H_2$ | 4,66 | d | $J_{2-3} = 5,8$ |
| $H_3$ | 4,84 | dd | $J_{3-4} = 3,6$ |
| $H_4$ | 4,01 | m | $J_{4-5} = 7,8$ |
| $H_5$ | 4,40 | m | $J_{5-6a} = 6,1$ |
| $H_{6a}$ | 4,09 | dd | $J_{6a-6b} = 8,7$ |
| $H_{6b}$ | 4,03 | m | $J_{5-6b} = 4,8$ |
| $CH_{3\ iso}$ | 1,45 | s | -- |
| $CH_{3\ iso}$ | 1,42 | s | -- |
| $CH_{3\ iso}$ | 1,34 | s | -- |
| $CH_{3\ iso}$ | 1,30 | s | -- |
| $H_{2'}$ | 2,62 | t | $J_{2'-3'} = 7,7$ |
| $H_{3'}$ | 1,92 | t | $J_{3'-4'} = 7,4$ |
| $H_{4'}$ | 2,29 | | -- |
| $H_{6'}$ | 7,29 | | -- |
| $H_{7'}$ | à | m | -- |
| $H_{8'}$ | 7,13 | | -- |

(Solvant : $CDCl_3$ ; déplacement chimique δ par rapport au signal du TMS).

Tableau 6 : Spectres RMN $^1H$ et $^{13}C$ du O-*n*-butanoyl-2,3-O-isopropylidène-α-D-mannoside.

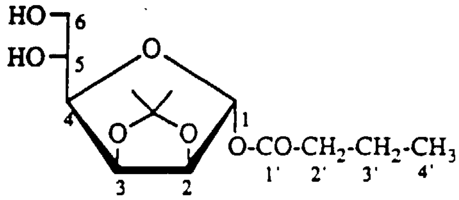

| C | δ (ppm) |
|---|---|
| $C_1$ | 99,4 |
| $C_2$ | 83,7 |
| $C_3$ | 78,7 |
| $C_4$ | 80,3 |
| $C_5$ | 68,8 |
| $C_6$ | 63,1 |
| $C_{iso}$ | 112,2 |
| $CH_3$ | 24,9 |
| $CH_3$ | 23,7 |
| $C_{1'}$ | 171,1 |
| $C_{2'}$ | 35,1 |
| $C_{3'}$ | 17,2 |
| $C_{4'}$ | 12,5 |

| H | δ (ppm) | | J (Hz) |
|---|---|---|---|
| $H_1$ | 6,05 | s | $J_{1-2} = 0$ |
| $H_2$ | 4,58 | d | $J_{2-3} = 5,9$ |
| $H_3$ | 4,81 | dd | $J_{3-4} = 3,4$ |
| $H_4$ | 3,94 | dd | $J_{4-5} = 8,5$ |
| $H_5$ | 3,91 | m | $J_{5-6a} = 7,9$ |
| $H_{6a}$ | 3,73 | dd | $J_{6a-6b}$ = n.d. |
| $H_{6b}$ | 3,59 | dd | $J_{5-6b}$ = n.d. |
| $CH_{3\ iso}$ | 1,38 | s | -- |
| $CH_{3\ iso}$ | 1,24 | s | -- |
| $H_{2'}$ | 2,17 | t | $J_{2'-3'} = 7,4$ |
| $H_{3'}$ | 1,53 | q | $J_{3'-4'} = 7,4$ |
| $H_{4'}$ | 0,83 | t | -- |
| OH | 3,33 | s | -- |
| OH | 2,85 | s | -- |

(Solvant : $CDCl_3$ ; déplacement chimique δ par rapport au signal du TMS).

Tableau 7 : Spectres RMN $^{1}H$ et $^{13}C$ du O-phénylacétyl-2,3-O-isopropylidène-α-D-mannoside.

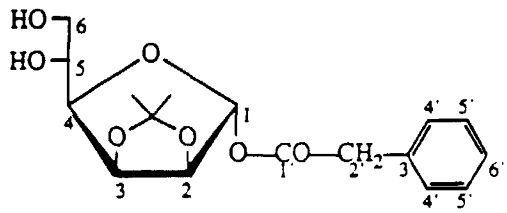

| C | δ (ppm) |
|---|---|
| $C_1$ | 100,6 |
| $C_2$ | 84,6 |
| $C_3$ | 79,6 |
| $C_4$ | 81,3 |
| $C_5$ | 69,8 |
| $C_6$ | 64,1 |
| $C_{iso}$ | 113,1 |
| $CH_3$ | 26,0 |
| $CH_3$ | 24,7 |
| $C_{1'}$ | 171,5 |
| $C_{2'}$ | 41,3 |
| $C_{3'}$ | 133,7 |
| $C_{4'}$ | 128,4 |
| $C_{5'}$ | 128,2 |
| $C_{6'}$ | 125,9 |

| H | δ (ppm) | | J (Hz) |
|---|---|---|---|
| $H_1$ | 6,15 | s | $J_{1-2} = 0$ |
| $H_2$ | 4,55 | d | $J_{2-3} = 5,9$ |
| $H_3$ | 4,80 | dd | $J_{3-4} = 2,5$ |
| $H_4$ | 4,00 | m | $J_{4-5} = 7,7$ |
| $H_5$ | 3,95 | m | $J_{5-6a} = 2,4$ |
| $H_{6a}$ | 3,83 | dd | $J_{6a-6b} = 11,5$ |
| $H_{6b}$ | 3,62 | m | $J_{5-6b} = 4,7$ |
| $CH_{3\ iso}$ | 1,45 | s | -- |
| $CH_{3\ iso}$ | 1,28 | s | -- |
| $H_{2'}$ | 2,92 | s | -- |
| $H_{4'}$ | 7,25 | | -- |
| $H_{5'}$ | à | m | -- |
| $H_{6'}$ | 7,12 | | -- |
| $OH_{5,6}$ | 2,80 | m | -- |

(Solvant : $CDCl_3$ ; déplacement chimique δ par rapport au signal du TMS).

Tableau 8 : Spectres RMN $^1H$ et $^{13}C$ du O-3'-phénylpropanoyl-2,3-O-isopropylidène-α-D-mannoside.

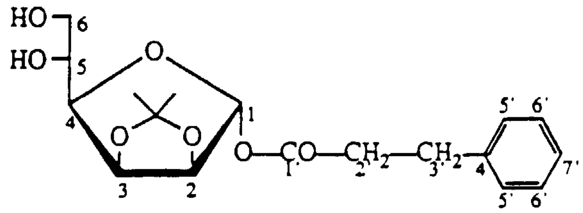

| C | δ (ppm) |
|---|---|
| $C_1$ | 100,6 |
| $C_2$ | 84,7 |
| $C_3$ | 79,7 |
| $C_4$ | 81,3 |
| $C_5$ | 69,9 |
| $C_6$ | 64,0 |
| $C_{iso}$ | 113,2 |
| $CH_3$ | 25,9 |
| $CH_3$ | 24,7 |
| $C_{1'}$ | 171,1 |
| $C_{2'}$ | 35,7 |
| $C_{3'}$ | 30,6 |
| $C_{4'}$ | 140,0 |
| $C_{5'}$ | 128,4 |
| $C_{6'}$ | 128,2 |
| $C_{7'}$ | 126,3 |

| H | δ (ppm) | | J (Hz) |
|---|---|---|---|
| $H_1$ | 6,10 | s | $J_{1-2} = 0$ |
| $H_2$ | 4,50 | d | $J_{2-3} = 5,8$ |
| $H_3$ | 4,77 | dd | $J_{3-4} = 2,6$ |
| $H_4$ | 3,94 | m | $J_{4-5} = 7,9$ |
| $H_5$ | 3,91 | m | $J_{5-6a} = 2,3$ |
| $H_{6a}$ | 3,77 | dd | $J_{6a-6b} = 11,5$ |
| $H_{6b}$ | 3,61 | m | $J_{5-6b} = 4,6$ |
| $CH_{3\ iso}$ | 1,44 | s | -- |
| $CH_{3\ iso}$ | 1,29 | s | -- |
| $H_{2'}$ | 2,90 | t | $J_{2'-3'} = 7,5$ |
| $H_{3'}$ | 2,60 | t | -- |
| $H_{5'}$ | 7,27 | | -- |
| $H_{6'}$ | à | m | -- |
| $H_{7'}$ | 7,03 | | -- |
| $OH_{5,6}$ | 3,15 | m | -- |

(Solvant : $CDCl_3$ ; déplacement chimique δ par rapport au signal du TMS).

Tableau 9 : Spectres RMN $^1H$ et $^{13}C$ du O-4'-phénylbutanoyl-2,3-O-isopropylidène-α-D-mannoside.

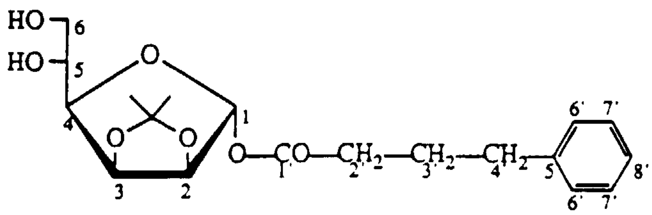

| C | δ (ppm) |
|---|---|
| $C_1$ | 100,5 |
| $C_2$ | 84,8 |
| $C_3$ | 79,7 |
| $C_4$ | 81,3 |
| $C_5$ | 69,9 |
| $C_6$ | 64,0 |
| $C_{iso}$ | 113,2 |
| $CH_3$ | 26,0 |
| $CH_3$ | 24,7 |
| $C_{1'}$ | 171,7 |
| $C_{2'}$ | 34,9 |
| $C_{3'}$ | 33,4 |
| $C_{4'}$ | 25,9 |
| $C_{5'}$ | 141,0 |
| $C_{6'}$ | 128,3 |
| $C_{7'}$ | 128,3 |
| $C_{8'}$ | 126,0 |

| H | δ (ppm) | | J (Hz) |
|---|---|---|---|
| $H_1$ | 6,16 | s | $J_{1\text{-}2} = 0$ |
| $H_2$ | 4,64 | d | $J_{2\text{-}3} = 5,9$ |
| $H_3$ | 4,86 | dd | $J_{3\text{-}4} = 3,5$ |
| $H_4$ | 4,03 | m | $J_{4\text{-}5} = 8,3$ |
| $H_5$ | 3,98 | m | $J_{5\text{-}6a} = 2,8$ |
| $H_{6a}$ | 3,81 | dd | $J_{6a\text{-}6b} = 11,5$ |
| $H_{6b}$ | 4,62 | m | $J_{5\text{-}6b} = 5,3$ |
| $CH_{3\ iso}$ | 1,45 | s | -- |
| $CH_{3\ iso}$ | 1,30 | s | -- |
| $H_{2'}$ | 2,60 | t | $J_{2'\text{-}3'} = 7,5$ |
| $H_{3'}$ | 1,91 | t | $J_{3'\text{-}4'} = 7,4$ |
| $H_{4'}$ | 2,27 | | -- |
| $H_{6'}$ | 7,27 | | -- |
| $H_{7'}$ | à | m | -- |
| $H_{8'}$ | 7,11 | | -- |
| $OH_{5,6}$ | 3,35 | m | |

(Solvant : $CDCl_3$ ; déplacement chimique δ par rapport au signal du TMS).

Tableau 10 : Spectres RMN $^{1}H$ et $^{13}C$ du 1-O-*n*-butanoyl-2,3:4,5-di-O-isopropylidène-xylitol.

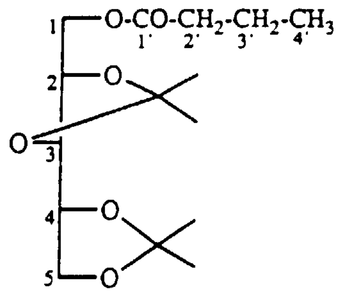

| C | δ (ppm) |
|---|---|
| $C_1$ | 63,9 |
| $C_2$ | 75,3 |
| $C_3$ | 77,4 |
| $C_4$ | 74,8 |
| $C_5$ | 65,4 |
| $C_{iso}$ | 109,9 |
| $C_{iso}$ | 109,6 |
| $CH_3$ | 26,9 |
| $CH_3$ | 26,8 |
| $CH_3$ | 26,0 |
| $CH_3$ | 25,2 |
| $C_{1'}$ | 173,0 |
| $C_{2'}$ | 35,8 |
| $C_{3'}$ | 18,2 |
| $C_{4'}$ | 13,4 |

| H | δ (ppm) | | J (Hz) |
|---|---|---|---|
| $H_{1a}$ | 4,28 | dd | $J_{1a\text{-}21b}$= 11,0 |
| $H_{1b}$ | 4,07 | dd | $J_{1a\text{-}2}$= 2,6<br>$J_{1b\text{-}2}$= 5,5 |
| $H_2$ | 4,12 | m | $J_{2\text{-}3}$ = 7,3 |
| $H_3$ | 3,85 | dd | $J_{3\text{-}4}$ = 4,5 |
| $H_4$ | 4,18 | ddd | $J_{4\text{-}5a}$ = 8,0 |
| $H_{5a}$ | 4,03 | dd | $J_{5a\text{-}5b}$ = 7,7 |
| $H_{5b}$ | 3,83 | dd | $J_{4\text{-}5b}$= 6,7 |
| $CH_{3\ iso}$ | 1,37 | s | -- |
| $CH_{3\ iso}$ | 1,35 | s | -- |
| $CH_{3\ iso}$ | 1,35 | s | -- |
| $CH_{3\ iso}$ | 1,30 | s | -- |
| $H_{2'}$ | 2,28 | t | $J_{2'\text{-}3'}$= 7,4 |
| $H_{3'}$ | 1,55 | q | $J_{3'\text{-}4'}$= 7,3 |
| $H_{4'}$ | 0,88 | t | -- |

(Solvant : $CDCl_3$ ; déplacement chimique δ par rapport au signal du TMS).

Tableau 11 : Spectres RMN $^1H$ et $^{13}C$ du 1-O-phénylacétyl-2,3:4,5-di-O-isopropylidène-xylitol.

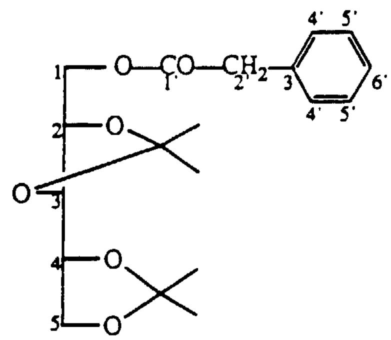

| C | δ (ppm) |
|---|---|
| $C_1$ | 64,4 |
| $C_2$ | 77,4 |
| $C_3$ | 75,1 |
| $C_4$ | 74,7 |
| $C_5$ | 65,5 |
| $C_{iso}$ | 110,0 |
| $C_{iso}$ | 109,7 |
| $CH_3$ | 26,9 |
| $CH_3$ | 26,8 |
| $CH_3$ | 26,0 |
| $CH_3$ | 25,3 |
| $C_{1'}$ | 171,0 |
| $C_{2'}$ | 41,1 |
| $C_{3'}$ | 133,6 |
| $C_{4'}$ | 129,2 |
| $C_{5'}$ | 128,5 |
| $C_{6'}$ | 127,1 |

| H | δ (ppm) | | J (Hz) |
|---|---|---|---|
| $H_{1a}$ | 4,21 | dd | $J_{1a-21b}$= 11,0 |
| $H_{1b}$ | 4,01 | dd | $J_{1a-2}$= 2,5<br>$J_{1b-2}$= 5,7 |
| $H_2$ | 4,04 | m | $J_{2-3}$ = 7,3 |
| $H_3$ | 3,71 | dd | $J_{3-4}$ = 3,9 |
| $H_4$ | 4,08 | ddd | $J_{4-5a}$ = 8,1 |
| $H_{5a}$ | 3,88 | dd | $J_{5a-5b}$ = 7,2 |
| $H_{5b}$ | 3,73 | dd | $J_{4-5b}$= 6,7 |
| $CH_3$ iso | 1,38 | s | -- |
| $CH_3$ iso | 1,36 | s | -- |
| $CH_3$ iso | 1,33 | s | -- |
| $CH_3$ iso | 1,32 | s | -- |
| $H_{2'}$ | 2,11 | s | -- |
| $H_{4'}$ | 7,29 | | -- |
| $H_{5'}$ | à | m | -- |
| $H_{6'}$ | 7,15 | | -- |

(Solvant : $CDCl_3$ ; déplacement chimique δ par rapport au signal du TMS).

Tableau 12 : Spectres RMN $^{1}H$ et $^{13}C$ du 1-O-3'-phénylpropanoyl-2,3:4,5-di-O-isopropylidène-xylitol.

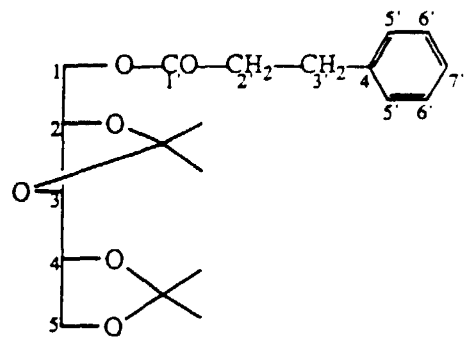

| C | δ (ppm) |
|---|---|
| $C_1$ | 64,1 |
| $C_2$ | 77,3 |
| $C_3$ | 75,2 |
| $C_4$ | 74,8 |
| $C_5$ | 65,4 |
| $C_{iso}$ | 110,0 |
| $C_{iso}$ | 109,7 |
| $CH_3$ | 26,9 |
| $CH_3$ | 26,9 |
| $CH_3$ | 26,0 |
| $CH_3$ | 25,3 |
| $C_{1'}$ | 172,4 |
| $C_{2'}$ | 35,6 |
| $C_{3'}$ | 30,8 |
| $C_{4'}$ | 140,2 |
| $C_{5'}$ | 128,4 |
| $C_{6'}$ | 128,1 |
| $C_7$ | 126,2 |

| H | δ (ppm) | | J (Hz) |
|---|---|---|---|
| $H_{1a}$ | 4,24 | dd | $J_{1a-21b}$= 11,3 |
| $H_{1b}$ | 4,08 | dd | $J_{1a-2}$= 2,7<br>$J_{1b-2}$= 6,5 |
| $H_2$ | 4,10 | m | $J_{2-3}$ = 7,9 |
| $H_3$ | 3,78 | dd | $J_{3-4}$ = 4,2 |
| $H_4$ | 4,13 | ddd | $J_{4-5a}$ = 8,1 |
| $H_{5a}$ | 3,99 | dd | $J_{5a-5b}$ = 7,1 |
| $H_{5b}$ | 3,79 | dd | $J_{4-5b}$= 6,7 |
| $CH_{3\ iso}$ | 1,37 | s | -- |
| $CH_{3\ iso}$ | 1,36 | s | -- |
| $CH_{3\ iso}$ | 1,35 | s | -- |
| $CH_{3\ iso}$ | 1,31 | s | -- |
| $H_{2'}$ | 2,92 | t | $J_{2'-3'}$= 7,6 |
| $H_{3'}$ | 2,65 | q | -- |
| $H_{5'}$ | 7,27 | t | -- |
| $H_{6'}$ | à | q | -- |
| $H_{7'}$ | 7,14 | t | -- |

(Solvant : $CDCl_3$ ; déplacement chimique δ par rapport au signal du TMS).

Tableau 13 : Spectres RMN $^{1}H$ et $^{13}C$ du 1-O-4'-phénylbutanoyl-2,3:4,5-di-O-isopropylidène-xylitol.

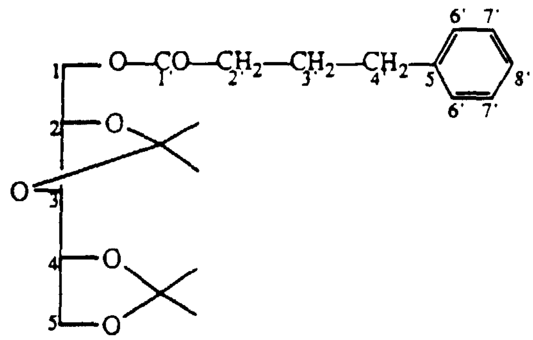

| C | δ (ppm) |
|---|---|
| $C_1$ | 64,1 |
| $C_2$ | 77,4 |
| $C_3$ | 75,3 |
| $C_4$ | 74,8 |
| $C_5$ | 65,5 |
| $C_{iso}$ | 110,0 |
| $C_{iso}$ | 109,7 |
| $CH_3$ | 26,9 |
| $CH_3$ | 26,3 |
| $CH_3$ | 26,0 |
| $CH_3$ | 25,3 |
| $C_{1'}$ | 172,9 |
| $C_{2'}$ | 35,0 |
| $C_{3'}$ | 33,3 |
| $C_{4'}$ | 26,9 |
| $C_{5'}$ | 141,1 |
| $C_{6'}$ | 128,3 |
| $C_{7'}$ | 128,3 |
| $C_{8'}$ | 125,9 |

| H | δ (ppm) | | J (Hz) |
|---|---|---|---|
| $H_{1a}$ | 4,26 | dd | $J_{1a-21b}$= 11,2 |
| $H_{1b}$ | 4,07 | dd | $J_{1a-2}$= 2,8<br>$J_{1b-2}$= 5,7 |
| $H_2$ | 4,13 | m | $J_{2-3}$ = 8,0 |
| $H_3$ | 3,84 | dd | $J_{3-4}$ = 4,4 |
| $H_4$ | 4,16 | ddd | $J_{4-5a}$ = 5,7 |
| $H_{5a}$ | 4,02 | dd | $J_{5a-5b}$ = 7,8 |
| $H_{5b}$ | 3,83 | dd | $J_{4-5b}$= 6,7 |
| $CH_{3\ iso}$ | 1,40 | s | -- |
| $CH_{3\ iso}$ | 1,38 | s | -- |
| $CH_{3\ iso}$ | 1,37 | s | -- |
| $CH_{3\ iso}$ | 1,34 | s | -- |
| $H_{2'}$ | 2,62 | t | $J_{2'-3'}$= 7,6 |
| $H_{3'}$ | 1,93 | q | $J_{3'-4'}$= 7,5 |
| $H_{4'}$ | 2,34 | t | -- |
| $H_6$ | 7,27 | | -- |
| $H_{7'}$ | à | m | -- |
| $H_{8'}$ | 7,12 | | -- |

(Solvant : $CDCl_3$ ; déplacement chimique δ par rapport au signal du TMS).

Tableau 14 : Spectres RMN $^{1}H$ et $^{13}C$ du 1-O-*n*-butanoyl-2,3-O-isopropylidène-xylitol.

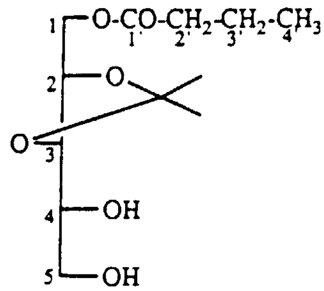

| C | δ (ppm) |
|---|---|
| $C_1$ | 65,8 |
| $C_2$ | 77,2 |
| $C_3$ | 77,3 |
| $C_4$ | 68,3 |
| $C_5$ | 64,7 |
| $C_{iso}$ | 109,5 |
| $CH_3$ | 26,1 |
| $CH_3$ | 25,9 |
| $C_{1'}$ | 172,3 |
| $C_{2'}$ | 35,0 |
| $C_{3'}$ | 17,3 |
| $C_{4'}$ | 12,6 |

| H | δ (ppm) | | J (Hz) |
|---|---|---|---|
| $H_{1a}$ | 4,26 | dd | $J_{1a-21b}$= 11,6 |
| $H_{1b}$ | 4,08 | dd | $J_{1a-2}$= 3,5<br>$J_{1b-2}$= 4,3 |
| $H_2$ | 4,20 | ddd | $J_{2-3}$ = 7,9 |
| $H_3$ | 3,83 | dd | $J_{3-4}$ = 3,0 |
| $H_4$ | 3,65 | ddd | $J_{4-5a}$ = 3,9 |
| $H_{5a}$ | 3,74 | dd | $J_{5a-5b}$ = 12,0 |
| $H_{5b}$ | 3,61 | dd | $J_{4-5b}$= 4,0 |
| $CH_3$ iso | 1,37 | s | -- |
| $CH_3$ iso | 1,36 | s | -- |
| $H_{2'}$ | 2,30 | t | $J_{2'-3'}$= 7,3 |
| $H_{3'}$ | 1,60 | q | $J_{3'-4'}$= 6,3 |
| $H_{4'}$ | 0,89 | t | -- |
| $OH_{4,5}$ | 2,56 | s | -- |

(Solvant : $CDCl_3$ ; déplacement chimique δ par rapport au signal du TMS).

Tableau 15 : Spectres RMN $^1H$ et $^{13}C$ du 1-O-*n*-butanoyl-xylitol.

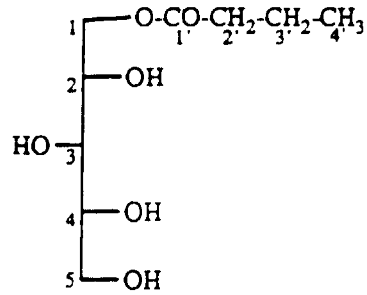

| C | δ (ppm) |
|---|---|
| $C_1$ | 66,9 |
| $C_2$ | 72,3 |
| $C_3$ | 74,0 |
| $C_4$ | 71,4 |
| $C_5$ | 64,4 |
| $C_{1'}$ | 173,0 |
| $C_{2'}$ | 36,2 |
| $C_{3'}$ | 18,7 |
| $C_{4'}$ | 13,7 |

| H | δ (ppm) | | J (Hz) |
|---|---|---|---|
| $H_{1a}$ | 4,52 | dd | $J_{1a-21b}$= 11,1 |
| $H_{1b}$ | 4,47 | dd | $J_{1a-2}$= 6,7<br>$J_{1b-2}$= 4,7 |
| $H_2$ | 4,36 | ddd | $J_{2-3}$ = 3,8 |
| $H_3$ | 4,25 | dd | $J_{3-4}$ = 5,5 |
| $H_4$ | 4,21 | m | $J_{4-5a}$ = 3,6 |
| $H_{5a}$ | 4,09 | dd | $J_{5a-5b}$ = n.d. |
| $H_{5b}$ | 4,09 | dd | $J_{4-5b}$= 6,0 |
| $H_{2'}$ | 1,88 | t | $J_{2'-3'}$= 7,3 |
| $H_{3'}$ | 1,22 | q | $J_{3'-4'}$= 7,3 |
| $H_{4'}$ | 0,44 | t | -- |
| $OH_{2-5}$ | 5,5-6,4 | m | -- |

(Solvant : $C_5$ $D_5N$; déplacement chimique δ par rapport au signal du TMS).

Tableau 16 : Spectres RMN $^1H$ et $^{13}C$ du 1-O-phénylacétyl-2,3-O-isopropylidène-xylitol.

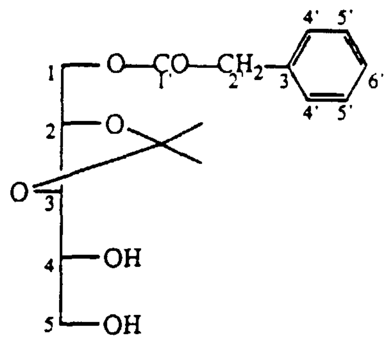

| C | δ (ppm) | H | δ (ppm) | | J (Hz) |
|---|---|---|---|---|---|
| $C_1$ | 64,5 | $H_{1a}$ | 4,28 | dd | $J_{1a\text{-}21b}$= 11,5 |
| $C_2$ | 74,9 | $H_{1b}$ | 4,12 | dd | $J_{1a\text{-}2}$= 3,5<br>$J_{1b\text{-}2}$= 4,5 |
| $C_3$ | 78,9 | $H_2$ | 4,19 | m | $J_{2\text{-}3}$ = 7,9 |
| $C_4$ | 69,7 | $H_3$ | 3,78 | dd | $J_{3\text{-}4}$ = 2,6 |
| $C_5$ | 64,0 | $H_4$ | 3,63 | ddd | $J_{4\text{-}5a}$ = n.d. |
| $C_{iso}$ | 109,9 | $H_{5a}$ | 3,60 | dd | $J_{5a\text{-}5b}$ = 8,0 |
| $CH_3$ | 26,9 | $H_{5b}$ | 3,53 | dd | $J_{4\text{-}5b}$= 3,9 |
| $CH_3$ | 26,8 | $CH_{3\ iso}$ | 1,33 | s | -- |
| $C_{1'}$ | 171,2 | $CH_{3\ iso}$ | 1,35 | s | -- |
| $C_{2'}$ | 41,1 | $H_{2'}$ | 2,37 | s | -- |
| $C_{3'}$ | 133,6 | $H_{4'}$ | 7,30 | | -- |
| $C_{4'}$ | 129,2 | $H_{5'}$ | à | m | -- |
| $C_{5'}$ | 128,5 | $H_{6'}$ | 7,22 | | -- |
| $C_{6'}$ | 127,1 | $OH_{4,5}$ | 2,7 | m | -- |

(Solvant : $CDCl_3$ ; déplacement chimique δ par rapport au signal du TMS).

Tableau 17 : Spectres RMN $^{1}H$ et $^{13}C$ du 1-O-phénylacétyl-xylitol.

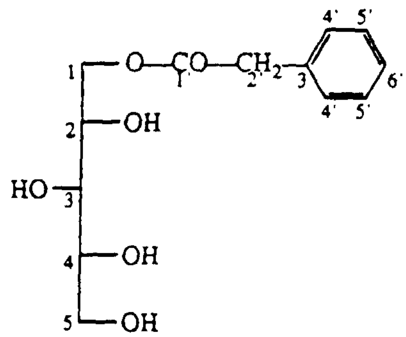

| C | δ (ppm) |
|---|---|
| $C_1$ | 67,8 |
| $C_2$ | 72,6 |
| $C_3$ | 74,2 |
| $C_4$ | 71,6 |
| $C_5$ | 64,8 |
| $C_{1'}$ | 172,1 |
| $C_{2'}$ | 41,7 |
| $C_{3'}$ | 135,4 |
| $C_{4'}$ | 130,2 |
| $C_{5'}$ | 129,2 |
| $C_{6'}$ | 127,6 |

| H | δ (ppm) | | J (Hz) |
|---|---|---|---|
| $H_{1a}$ | 4,75 | dd | $J_{1a-21b}$= 11,2 |
| $H_{1b}$ | 4,70 | dd | $J_{1a-2}$= 7,3<br>$J_{1b-2}$= 4,8 |
| $H_2$ | 4,56+ | m | $J_{2-3}$ = 3,7 |
| $H_3$ | 4,43 | dd | $J_{3-4}$ = 4,8 |
| $H_4$ | 4,40 | ddd | $J_{4-5a}$ = 3,1 |
| $H_{5a}$ | 4,26 | dd | $J_{5a-5b}$ = n.d. |
| $H_{5b}$ | 4,21 | dd | $J_{4-5b}$= 5,1 |
| $H_{2'}$ | 1,94 | s | -- |
| $H_{4'}$ | 7,33 | | -- |
| $H_{5'}$ | à | m | -- |
| $H_{6'}$ | 7,13 | | -- |
| $OH_{2,5}$ | 6,11 | m | -- |

(Solvant : $C_5D_5N$ ; déplacement chimique δ par rapport au signal du TMS).

Tableau 18 : Spectres RMN $^{1}H$ et $^{13}C$ du 1-O-3'-phénylpropanoyl-2,3-O-isopropylidène-xylitol.

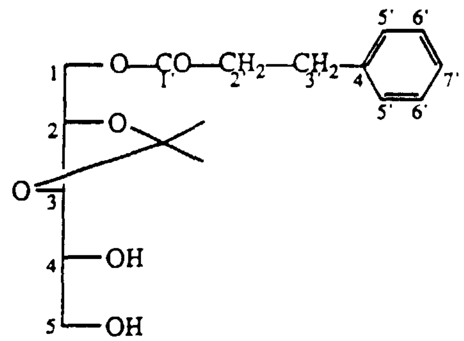

| C | δ (ppm) | H | δ (ppm) | | J (Hz) |
|---|---|---|---|---|---|
| $C_1$ | 64,5 | $H_{1a}$ | 4,29 | dd | $J_{1a-21b}$= 11,2 |
| $C_2$ | 75,1 | $H_{1b}$ | 4,16 | dd | $J_{1a-2}$= 3,6<br>$J_{1b-2}$= 1,4 |
| $C_3$ | 77,9 | $H_2$ | 4,23 | m | $J_{2-3}$ = 7,3 |
| $C_4$ | 69,8 | $H_3$ | 3,82 | dd | $J_{3-4}$ = 3,0 |
| $C_5$ | 63,7 | $H_4$ | 3,69 | ddd | $J_{4-5a}$ = 7,0 |
| $C_{iso}$ | 110,0 | $H_{5a}$ | 3,71 | dd | $J_{5a-5b}$ = 7,9 |
| $CH_3$ | 26,8 | $H_{5b}$ | 3,63 | dd | $J_{4-5b}$= 4,3 |
| $CH_3$ | 26,4 | $CH_{3\ iso}$ | 1,38 | s | -- |
| $C_{1'}$ | 172,4 | $CH_{3\ iso}$ | 1,36 | s | -- |
| $C_{2'}$ | 35,5 | $H_{2'}$ | 2,91 | t | $J_{2'-3'}$= 7,7 |
| $C_{3'}$ | 30,7 | $H_{3'}$ | 2,64 | t | -- |
| $C_{4'}$ | 140,2 | $H_{5'}$ | 7,27 | | -- |
| $C_{5'}$ | 128,4 | $H_{6'}$ | à | m | -- |
| $C_{6'}$ | 128,2 | $H_{7'}$ | 7,14 | | -- |
| $C_{7'}$ | 126,2 | $OH_{4,5}$ | 4,16 | m | -- |

(Solvant : $CDCl_3$ ; déplacement chimique δ par rapport au signal du TMS).

Tableau 19 : Spectres RMN $^1H$ et $^{13}C$ du 1-O-3'-phénylpropanoyl-xylitol.

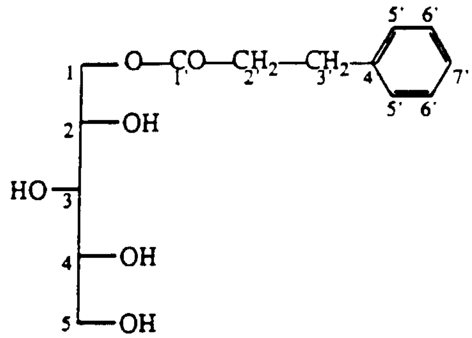

| C | δ (ppm) |
|---|---|
| $C_1$ | 67,4 |
| $C_2$ | 72,6 |
| $C_3$ | 74,3 |
| $C_4$ | 71,7 |
| $C_5$ | 64,8 |
| $C_{1'}$ | 173,3 |
| $C_{2'}$ | 36,3 |
| $C_{3'}$ | 30,9 |
| $C_{4'}$ | 141,6 |
| $C_{5'}$ | 129,2 |
| $C_{6'}$ | 129,0 |
| $C_{7'}$ | 126,9 |

| H | δ (ppm) | | J (Hz) |
|---|---|---|---|
| $H_{1a}$ | 4,66 | dd | $J_{1a-21b}$= 10,7 |
| $H_{1b}$ | 4,63 | dd | $J_{1a-2}$= 7,2<br>$J_{1b-2}$= 4,5 |
| $H_2$ | 4,49 | m | $J_{2-3}$ = 3,7 |
| $H_3$ | 4,38 | dd | $J_{3-4}$ = 4,9 |
| $H_4$ | 4,36 | ddd | $J_{4-5a}$ = 3,3 |
| $H_{5a}$ | 4,22 | dd | $J_{5a-5b}$ = n.d. |
| $H_{5b}$ | 4,18 | dd | $J_{4-5b}$= 5,6 |
| $H_{2'}$ | 2,87 | t | $J_{2'-3'}$= 7,7 |
| $H_{3'}$ | 2,60 | t | -- |
| $H_{5'}$ | 7,23 | | -- |
| $H_{6'}$ | à | m | -- |
| $H_{7'}$ | 7,09 | | -- |
| $OH_{2,5}$ | 6,04 | m | -- |

(Solvant : $C_5D_5N$; déplacement chimique δ par rapport au signal du TMS).

Tableau 20 : Spectres RMN $^1H$ et $^{13}C$ du 1-O-4'-phénylbutanoyl-2,3-O-isopropylidène-xylitol.

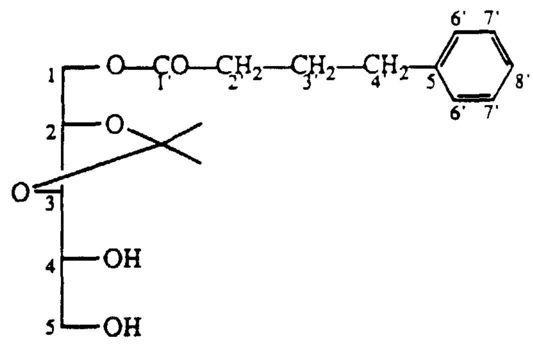

| C | δ (ppm) | H | δ (ppm) | | J (Hz) |
|---|---|---|---|---|---|
| $C_1$ | 64,4 | $H_{1a}$ | 4,44 | dd | $J_{1a-21b}$= 11,2 |
| $C_2$ | 75,2 | $H_{1b}$ | 4,10 | dd | $J_{1a-2}$= 3,5<br>$J_{1b-2}$= 5,3 |
| $C_3$ | 78,8 | $H_2$ | 4,21 | m | $J_{2-3}$ = 8,0 |
| $C_4$ | 70,0 | $H_3$ | 3,84 | dd | $J_{3-4}$ = 2,0 |
| $C_5$ | 63,8 | $H_4$ | 3,68 | ddd | $J_{4-5a}$ = n.d. |
| $C_{iso}$ | 110,0 | $H_{5a}$ | 3,68 | dd | $J_{5a-5b}$ = 3,2 |
| $CH_3$ | 26,8 | $H_{5b}$ | 3,65 | dd | $J_{4-5b}$ = n.d. |
| $CH_3$ | 26,2 | $CH_{3\ iso}$ | 1,38 | s | -- |
| $C_{1'}$ | 173,1 | $CH_{3\ iso}$ | 1,36 | s | -- |
| $C_{2'}$ | 35,0 | $H_{2'}$ | 2,60 | t | $J_{2'-3'}$= 7,5 |
| $C_{3'}$ | 33,3 | $H_{3'}$ | 1,93 | q | $J_{3'-4'}$= 7,6 |
| $C_{4'}$ | 26,9 | $H_{4'}$ | 2,32 | t | -- |
| $C_{5'}$ | 141,1 | $H_{6'}$ | 7,26 | | -- |
| $C_{6'}$ | 128,3 | $H_{7'}$ | à | m | -- |
| $C_7$ | 128,3 | $H_{8'}$ | 7,11 | | -- |
| $C_{8'}$ | 125,9 | $OH_{4,5}$ | 4,15 | m | -- |

(Solvant : $CDCl_3$ ; déplacement chimique δ par rapport au signal du TMS).

Tableau 21 : Spectres RMN $^1H$ et $^{13}C$ du 1-O-4'-phénylbutanoyl-xylitol.

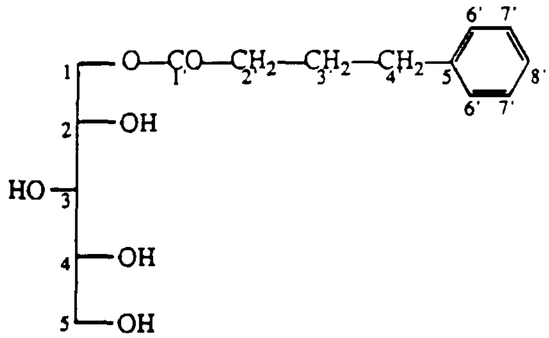

| C | δ (ppm) |
|---|---|
| $C_1$ | 67,3 |
| $C_2$ | 72,6 |
| $C_3$ | 74,3 |
| $C_4$ | 71,7 |
| $C_5$ | 64,8 |
| $C_{1'}$ | 173,9 |
| $C_{2'}$ | 35,6 |
| $C_{3'}$ | 34,6 |
| $C_{4'}$ | 27,3 |
| $C_{5'}$ | 142,4 |
| $C_{6'}$ | 129,2 |
| $C_{7'}$ | 129,1 |
| $C_{8'}$ | 126,6 |

| H | δ (ppm) | | J (Hz) |
|---|---|---|---|
| $H_{1a}$ | 4,75 | dd | $J_{1a\text{-}21b}$= 11,2 |
| $H_{1b}$ | 4,69 | dd | $J_{1a\text{-}2}$= 7,2<br>$J_{1b\text{-}2}$= 4,5 |
| $H_2$ | 4,56 | m | $J_{2\text{-}3}$ = 3,7 |
| $H_3$ | 4,43 | dd | $J_{3\text{-}4}$ = 5,0 |
| $H_4$ | 4,40 | ddd | $J_{4\text{-}5a}$ = 3,5 |
| $H_{5a}$ | 4,28 | dd | $J_{5a\text{-}5b}$ = n.d. |
| $H_{5b}$ | 4,22 | dd | $J_{4\text{-}5b}$= 5,4 |
| $H_{2'}$ | 2,52 | t | $J_{2'\text{-}3'}$= 7,7 |
| $H_{3'}$ | 1,87 | q | $J_{3'\text{-}4'}$= 7,0 |
| $H_{4'}$ | 2,30 | t | -- |
| $H_{6'}$ | 7,25 | | -- |
| $H_{7'}$ | à | m | -- |
| $H_{8'}$ | 7,10 | | -- |
| $OH_{2,5}$ | 5,92 | m | -- |

(Solvant : $C_5D_5N$ ; déplacement chimique δ par rapport au signal du TMS).

## Revendications

1. Mono-ester associant l'acide phénylacétique, 3-phénylpropionique, 4-phénylbutyrique ou n-butyrique à un ose ou un itol de formule $Su(OH)_5$, ledit monoester répondant à la formule générale suivante :

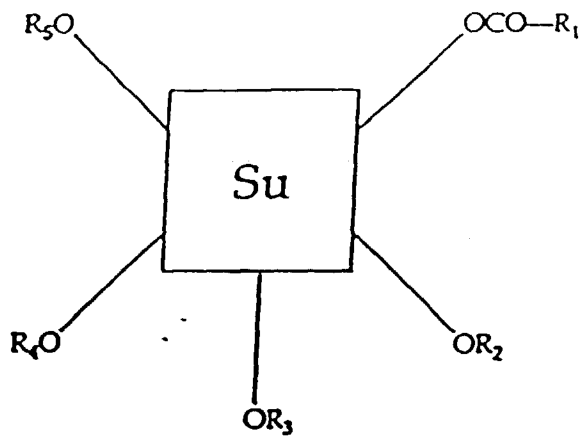

dans laquelle $R_1$-GO est le groupement acyle de l'acide phénylacétique, 3-phénylpropionique, 4-phénylbutyrique ou n-butyrique ;
dans laquelle $R_2$, $R_3$, $R_4$, $R_5$ peuvent être l'atome d'hydrogène ou des groupements comportant une chaîne hydrocarbonée, cycliques ou non, saturés ou non, ramifiés ou non, ou encore choisis de sorte que $R_2$ et $R_3$ ou $R_4$ et $R_5$ appartiennent à un groupement acétal sous forme de dioxolane :

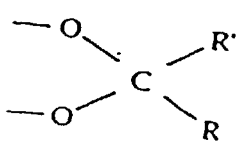

le groupement R-C-R' étant choisi parmi le phénylméthylène, le méthylène, le cyclohexylidène et l'isopropylidène
et au moins deux des groupes $R_2$, $R_3$, $R_4$ et $R_5$ représentant un atome d'hydrogène,
dans laquelle l'ose ou l'itol $Su(OH)_5$ porte respectivement le groupement ester sur l'atome de carbone anomérique ou sur l'atome de carbone primaire C-1 ;
la structure dudit mono-ester ne permettant pas le processus de transestérification interne, les groupes hydroxyles libres étant éloignés et/ou mal orientés par rapport au groupement ester, et/ou liés à un atome de carbone secondaire, tout en permettant une bonne biodisponibilité de l'acide correspondant.

**2.** Mono-ester, selon la revendication 1, caractérisé en ce que l'ose Su(OH)5 est le D-mannose.

**3.** Mono-ester selon la revendication 1, caractérisé en ce que l'itol $Su(OH)_5$ est le xylitol.

**4.** Mono-ester selon la revendication 2, caractérisé en ce qu'il consiste en l'α- ou le β-O-acyl-2,3-O-acétal-D-mannofuranoside ; ou en l'α- ou le β-O-acyl-D-mannofuranoside, le groupe acyle étant le groupe phénylacétyle, 3-phénylpropanoyle, 4-phénylbutanoyle ou n-butanoyle.

**5.** Mono-ester selon la revendication 3, caractérisé en ce qu'il est dérivé du xylitol et consiste en le 1-O-acyl-2,3-O-acétal-xylitol ; ou le 1-O-acylxylitol, le groupe acyle étant choisi parmi le groupe phénylacétyle, 3-phénylpropanoyle, 4-phénylbutanoyle ou n-butanoyle.

**6.** L'α-O-n-butanoyl-D-mannofuranoside et son dérivé α-O-n-butanoyl-2,3-O-isopropylidène-D-mannofuranoside.

**7.** L'α-O-phénylacétyl-D-mannofuranoside et son dérivé α-O-phénylacétyl-2,3-O-isopropylidène-D-mannofuranoside.

**8.** L'α-O-3'-phénylpropanoyl-D-mannofuranoside et son dérivé α-O-3-phénylpropanoyl-2,3-O-isopropylidène-D-mannofuranoside.

9. L'α-O-4'-phénylbutanoyl-D-mannofuranoside et son dérivé α-O-4'-phénylbutanoyl-2,3-O-isopopylidéne-D-mannofuranoside.

10. Le 1-O-n-butanoyl-xylitol et son dérivé 1-O-n-butanoyl-2,3-O-isopropylidène-xylitol.

11. Le 1-O-phénylacétyl-xylitol et son dérivé 1-O-phénylacétyl-2,3-O-isopropylidène-xylitol.

12. Le 1-O-3'-phénylpropanoyl-xylitol et son dérivé 1-O-3'-phénylpropanoyl-2,3-O-isopropylidéne-xylitol.

13. Le 1-O-4'-phénylbutanoyl-xylitol et son dérivé 1-O-4'-phénylbutanoyl-2,3-O-isopropylidène-xylitol.

14. Médicament destiné au traitement des hémoglobinopathies, comme les anémies, la β-thalassémie, la drépanocytose, comprenant un ester selon l'une quelconque des revendications 1 à 13.

15. Médicament destiné au traitement des tumeurs prémalignes et malignes, comme les cancers du sein, du colon, les leucémies aiguës et chroniques, comprenant un ester selon l'une quelconque des revendications 1 à 13.

16. Médicament selon l'une des revendications 14 et 15, caractérisé en ce qu'il comprend un ester dérivé du D-mannose.

17. Médicament selon l'une des revendications 14 et 15, caractérisé en ce qu'il comprend un ester dérivé du xylitol.

18. Utilisation d'un ester selon l'une quelconque des revendications 1 à 13, pour la préparation d'un médicament destiné au traitement des hémoglobinopathies, comme les anémies, la β-thalassémie, la drépanocytose.

19. Utilisation d'un ester selon l'une quelconque des revendications 1 à 13, pour la préparation d'un médicament destiné au traitement des tumeurs prémalignes et malignes, comme les cancers du sein, du colon, les leucémies aiguës et chroniques.

20. Utilisation selon l'une des revendications 18 et 19, caractérisée en ce que l'ester est dérivé du D-mannose.

21. Utilisation selon l'une des revendications 18 et 19, caractérisée en ce que l'ester est dérivé du xylitol.

## Patentansprüche

1. Monoester, der Phenylessigsäure, 3-Phenylpropionsäure, 4-Phenylbuttersäure oder n-Buttersäure an eine Ose oder ein Itol der Formel $Su(OH)_5$ bindet, wobei der Monoester der folgenden allgemeinen Formel entspricht:

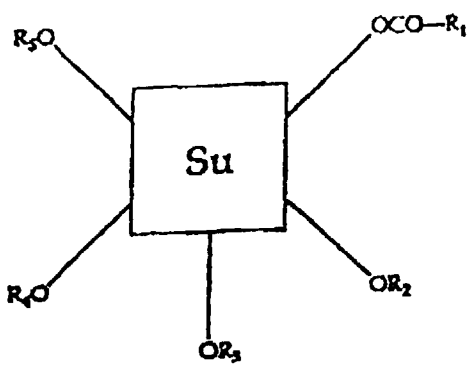

in der $R_1$-CO die Acylgruppe der Phenylessigsäure, 3-Phenylpropionsäure, 4-Phenylbuttersäure oder n-Buttersäure ist;
in der $R_2$, $R_3$, $R_4$ und $R_5$ ein Wasserstoffatom oder Gruppierungen sein können, die eine Kohlenwasserstoffkette tragen können oder nicht, die zyklisch oder nichtzyklisch, gesättigt oder ungesättigt, verzweigt oder unverzweigt ist, oder so ausgewählt sind, daß $R_2$ und $R_3$ oder $R_4$ und $R_5$ einer Acetalgruppe in Form eines

Dioxolans angehören:

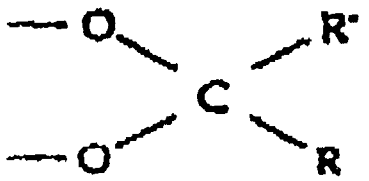

wobei die Gruppe R-C-R' ausgewählt ist aus Phenylmethylen, Methylen, Cyclohexyliden und Isopropyliden und mindestens zwei der Gruppen $R_2$, $R_3$, $R_4$ und $R_5$ ein Wasserstoffatom darstellen,
in der die Ose oder das Itol $Su(OH)_5$ die Estergruppierung jeweils am anomeren Kohlenstoffatom oder am primären C1-Atom trägt;
wobei die Struktur des Monoesters keinen internen Umesterungsprozeß zuläßt, die freien Hydroxylgruppen von der Estergruppe entfernt und/oder bezüglich dieser ungünstig orientiert sind und/oder an ein sekundäres Kohlenstoffatom gebunden sind, wodurch eine gute Bioverfügbarkeit der entsprechenden Säure ermöglicht wird.

**2.** Monoester nach Anspruch 1, dadurch gekennzeichnet, daß die Ose $Su(OH)_5$ D-Mannose ist.

**3.** Monoester nach Anspruch 1, dadurch gekennzeichnet, daß das Itol $Su(OH)_5$ Xylitol ist.

**4.** Monoester nach Anspruch 2, dadurch gekennzeichnet, daß er aus α- oder β-O-Acyl-2,3-O-acetal-D-mannofuranosid oder aus α- oder β-O-Acyl-D-mannofuranosid besteht, wobei die Acylgruppe eine Phenylacetyl-, 3-Phenylpropanoyl-, 4-Phenylbutanoyl- oder n-Butanoylgruppe ist.

**5.** Monoester nach Anspruch 3, dadurch gekennzeichnet, daß er von Xylitol herrührt und aus 1-O-Acyl-2,3-O-acetalxylitol oder 1-O-Acylxylitol besteht, wobei die Acylgruppe ausgewählt ist aus einer Phenylacetyl-, 3-Phenylpropanoyl-, 4-Phenylbutanoyl- oder n-Butanoylgruppe.

**6.** α-O-n-Butanoyl-D-mannofuranosid und sein Derivat α-O-n-Butanoyl-2,3-O-isopropyliden-D-mannofuranosid.

**7.** α-O-Phenylacetyl-D-mannofuranosid und sein Derivat α-O-Phenylacetyl-2,3-O-isopropyliden-D-mannofuranosid.

**8.** α-O-3'-Phenylpropanoyl-D-mannofuranosid und sein Derivat a-O-3'-Phenylpropanoyl-2,3-O-isopropyliden-D-mannofuranosid.

**9.** α-O-4'-Phenylbutanoyl-D-mannofuranosid und sein Derivat a-O-4'-Phenylbutanoyl-2,3-O-isopropyliden-D-mannofuranosid.

**10.** 1-O-n-Butanoylxylitol und sein Derivat 1-O-n-Butanoyl-2,3-O-isopropylidenxylitol.

**11.** 1-O-Phenylacetylxylitol und sein Derivat 1-O-Phenylacetyl-2,3-O-isopropylidenxylitol.

**12.** 1-O-3'-Phenylpropanoylxylitol und sein Derivat 1-O-3'-Phenylpropanoyl-2,3-O-isopropylidenxylitol.

**13.** 1-O-4'-Phenylbutanoylxylitol und sein Derivat 1-O-4'-Phenylbutanoyl-2,3-O-isopropylidenxylitol.

**14.** Medikament, das zur Behandlung von Hämoglobinopathien, wie Anämien, β-Thalassämie, Drepranozytose, bestimmt ist, umfassend einen Ester nach einem der Ansprüche 1 bis 13.

**15.** Medikament, das zur Behandlung von prämalignen und malignen Tumoren, wie Brust- und Colonkrebs, akuten und chronischen Leukämien, bestimmt ist, umfassend einen Ester nach einem der Ansprüche 1 bis 13.

**16.** Medikament nach einem der Ansprüche 14 und 15, dadurch gekennzeichnet, daß es einen von D-Mannose herrührenden Ester umfaßt.

**17.** Medikament nach einem der Ansprüche 14 und 15, dadurch gekennzeichnet, daß es einen von Xylitol herrührenden Ester umfaßt.

**18.** Verwendung eines Ester nach einem der Ansprüche 1 bis 13 zur Herstellung eines Medikamentes, das zur Behandlung von Hämoglobinopathien, wie Anämien, β-Thalassämie, Drepranozytose, bestimmt ist.

**19.** Verwendung eines Ester nach einem der Ansprüche 1 bis 13 zur Herstellung eines Medikamentes, das zur Behandlung von prämalignen und malignen Tumoren, wie Brust- und Colonkrebs, akuten und chronischen Leukämien, bestimmt ist.

**20.** Verwendung nach einem der Ansprüche 18 und 19, dadurch gekennzeichnet, daß der Ester von D-Mannose herrührt.

**21.** Verwendung nach einem der Ansprüche 18 und 19, dadurch gekennzeichnet, daß der Ester von Xylitol herrührt.

## Claims

**1.** Monoester associating phenylacetic, 3-phenylpropionic, 4-phenylbutyric or n-butyric acid with an ose or an itol of formula $Su(OH)_5$, the monoester corresponding to the following general formula:

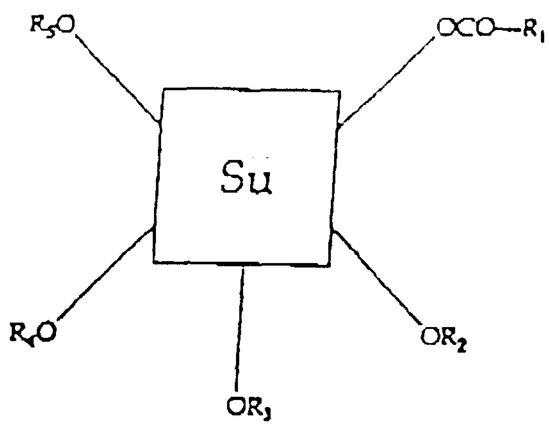

wherein $R_1$-CO is the acyl group of phenylacetic, β-phenylpropionic, 4-phenylbutyric or n-butyric acid;

wherein $R_2$, $R_3$, $R_4$, $R_5$ may be a hydrogen atom or cyclic or acyclic, saturated or unsaturated, branched or unbranched group comprising or not comprising a hydrocarbon chain, or selected in such a manner that $R_2$ and $R_3$ or $R_4$ and $R_5$ belong to an acetal group in the form of dioxolane:

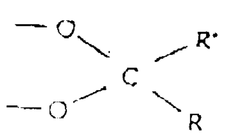

the group R-C-R' being selected from phenylmethylene, methylene, cyclohexylidene and isopropylidene,

and at least two of the groups $R_2$, $R_3$, $R_4$, and $R_5$ representing a hydrogen atom,

wherein the ose or itol $Su(OH)_5$ carries, respectively, the ester group on the anomeric carbon atom or on the primary carbon atom C-1;

the structure of the monoester not permitting an internal transesterification process, the free hydroxyl groups being at a distance from and/or poorly oriented relative to the ester group, and/or being linked to a secondary carbon atom, while at the same time parmitting good bioavailability of the corresponding acid.

**2.** Monoester according to claim 1, characterised in that the ose $Su(OH)_5$ is D-mannose.

**3.** Monoester according to claim 1, characterised in that the itol $Su(OH)_5$ is xylitol.

**4.** Monoester according to claim, 2, characterised in that it consists of α- or β-O-acyl-2,3-O-acetal-D-mannofuranoside, or of α- or β-O-acyl-D-mannofuranoside, the acyl group being the phenylacetyl, 3-phenylpropanoyl, 4-phenylbutanoyl or n-butanoyl group.

**5.** Monoester according to claim 3, characterised in that it is derived from xylitol and consists of 1-O-acyl-2,3-O-acetal xylitol or 1-O-acyl-xylitol the acyl group being selected from the phenylacetyl, β-phenylpropanoyl, 4-phenylbutanoyl or n-butanoyl group.

**6.** α-O-n-butanoyl-D-mannofuranoside and its derivative α-O-n-butanoyl-2,3-O isopropylidene-D-mannofuranoside.

**7.** α-O-phenylacetyl-D-mannofuranoside and its derivative α-O-phenylacetyl-2,3-O-isopropylidene-D-mannofuranoside.

**8.** α-O-3'-phenylpropanoyl-D-mannofuranoside and its derivative α-O-3-phenylpropanoyl-2,3-O-isopropylidene-D-mannofuranoside.

**9.** α-O-4'-phenylbutanoyl-D-mannofuranoside and its derivative α-O-4'-phenylbutanoyl-2,3-O-isopropylidene-D-mannofuranoside.

**10.** 1-O-n-butanoyl-xylitol and its derivative 1-O-n-butanoyl-2,3-O-isopropylidene-xylitol.

**11.** 1-O-pbenylacetyl-xylitol and its derivative 1-O-phenylacetyl-2,3-O-isopropylidene-xylitol.

**12.** 1-O-3'-phenylpropanoyl-xylitol and its derivative 1-O-3'-phenylpropanoyl-2,3-O-isopropylidene-xylitol.

**13.** 1-O-4'-phenylbutanoyl-xylitol and its derivative 1-O-4'-phenylbutanoyl-2,3-O-isopropylidene-xylitol.

**14.** Medicament for the treatment of haemoglobin diseases, such as anaemias, β-thalassaemia, drepanocytosis, comprising an ester according to any one of claims 1 to 13.

**15.** Medicament for the treatment of premalignant and malignant tumours, such as cancers of the breast, of the colon, acute and chronic leukaemias, comprising an ester according to any one of claims 1 to 13.

**16.** Medicament according to either claim 14 or claim 15, characterised in that it comprises an ester derived from D-mannose.

**17.** Medicament according to either claim 14 or claim 15, characterised in that it comprises an ester derived from xylitol.

**18.** Use of an ester according to any one of claims 1 to 13, for the preparation of a medicament for the treatment of haemoglobin diseases, such as anaemias, β-thalassaemia, drepanocytosis.

**19.** Use of an ester according to any one of claims 1 to 13 for the preparation of a medicament for the treatment of premalignant and malignant tumours, such as cancers of the breast, of the colon, acute and chronic leukaemias.

**20.** Use according to either claim 18 or claim 19, characterised in that the ester is derived from D-mannose.

**21.** Use according to either claim 18 or claim 19, characterised in than the ester is derived from xylitol.